(19) Europäisches Patentamt
European Patent Office
Office européen des brevets

(11) Numéro de publication : **0 487 603 B1**

# (12) FASCICULE DE BREVET EUROPEEN

(45) Date de publication du fascicule du brevet : **25.08.93 Bulletin 93/34**

(21) Numéro de dépôt : **90912897.7**

(22) Date de dépôt : **14.08.90**

(86) Numéro de dépôt international : **PCT/FR90/00611**

(87) Numéro de publication internationale : **WO 91/02525 07.03.91 Gazette 91/06**

(51) Int. Cl.$^5$ : **A61K 31/35**, A61K 35/78, A61K 9/127, A61K 7/00, A61K 7/48, C07D 311/92

## (54) COMPOSITION A BASE DE PHASES LAMELLAIRES LIPIDIQUES HYDRATEES OU DE LIPOSOMES CONTENANT AU MOINS UN DERIVE DE LABDANE, OU UN EXTRAIT DE PLANTE EN CONTENANT; COMPOSITION COSMETIQUE OU PHARMACEUTIQUE, NOTAMMENT DERMATOLOGIQUE L'INCORPORANT.

(30) Priorité : **17.08.89 FR 8910986**

(43) Date de publication de la demande : **03.06.92 Bulletin 92/23**

(45) Mention de la délivrance du brevet : **25.08.93 Bulletin 93/34**

(84) Etats contractants désignés : **AT BE CH DE DK ES GB IT LI LU NL SE**

(56) Documents cités :
**EP-A- 0 296 923**
**EP-A- 0 311 032**

(73) Titulaire : **LVMH RECHERCHE**
**48-50, rue de Seine, B.P. 79**
**F-92703 Colombes Cédex (FR)**

(72) Inventeur : **BONTE, Frédéric**
**5, place Charras**
**F-92400 Courbevoie (FR)**
Inventeur : **MEYBECK, Alain**
**Les Poissons 20, ter, rue de Bezons**
**F-92400 Courbevoie (FR)**
Inventeur : **MARECHAL, Christian**
**7, rue Charlot**
**F-75003 Paris (FR)**

(74) Mandataire : **Portal, Gérard et al**
**Cabinet Beau de Loménie 158, rue de l'Université**
**F-75340 Paris Cédex 07 (FR)**

EP 0 487 603 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

La présente invention concerne des compositions à base de phases lamellaires lipidiques hydratées ou de liposomes contenant au moins un dérivé de labdane, en particulier un labd-14-ène, ou un extrait végétal en contenant, destinées à la préparation de compositions cosmétiques ou pharmaceutiques, notamment dermatologiques.

Les labdanes, et en particulier leurs dérivés insaturés en position 14 dénommés labd-14-ènes, sont des diterpènes assez largement répandus dans la nature.

On les trouve en particulier dans différentes variétés de résineux (notamment les pins), tabacs, cistes, (en particulier Cistus ladaniferus, Cistus laurifolius), et Coleus (notamment Coleus forskohlii).

Le Coleus forskohlii représente l'une des quelques deux cents espèces connues de Coleus, appartenant à la famille des labiacées, et que l'on trouve dans les régions tropicales et subtropicales d'Asie, d'Afrique, d'Australie et des îles pacifiques. Environ neuf espèces sont répertoriées en Inde. Les dix variétés principales de Coleus sont répertoriées dans des dictionnaires indiens et en particulier "The Wealth of India, a dictionary of Indian Raw Materials and Industrial Products, Raw Materials, volume II, Dehli 1950, pages 308-309 ; le livre intitulé Indian Materia Medica du Docteur K.M. Nadkarni's, troisième édition révisée et complétée par A.K. Nadkarni en deux volumes, volume I, page 372 ; le livre intitulé "The Flora of British India par Sir J.D. Hooker, C.B., K.C., S.I. Volume IV ayant pour titre "Asclepiadae to Amarantaceae, pages 624 à 627.

Les dérivés de labdane auxquels se réfère la présente invention sont en particulier des dérivés de formule générale :

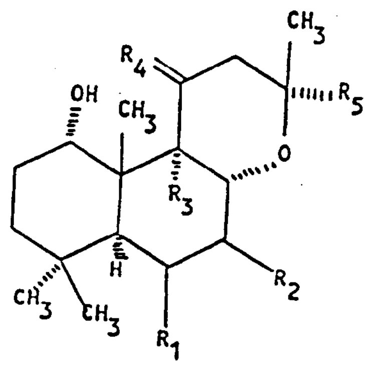

(I)

dans laquelle :

- $R_1$ à $R_2$ sont identiques ou différents et représentent chacun un groupe hydroxy ou un groupe -O-COR$_6$ dans lequel $R_6$ représente un radical alcoyle, alcoxy ou alcényle éventuellement substitué, notamment par un ou plusieurs groupes hydroxy ou amino
- $R_3$ représente un atome d'hydrogène ou un groupe hydroxy
- $R_4$ représente un atome d'oxygéne ou l'ensemble

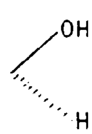

- $R_6$ représente un radical éthyle ou vinyle

Plusieurs dérivés de labdane répondant à la formule précitée ont été extraits de diverses plantes et notamment de la plante Coleus forskolii. On se reportera en particulier aux documents FR-2 336 138, FR-2 364 211 et EP-A-1-0 243 646 qui décrivent la forskoline (composé de formule I dans laquelle $R_1 = R_3 = OH$ ; $R_2 = OCOCH_3$ ; $R_4 = O$ ; $R_6 = CH = CH_2$) la 9-déoxy forskoline (composé de formule I dans laquelle $R_1 = OH$, $R_2 = OCOCH_3$; $R_3 = H$ ; $R_4 = O$ ; $R_5 = CH = CH_2$) et la coléforsine (composé de formule I dans laquelle $R_2 = R_3 = OH$ ; $R_1 = OCOCH_3$ ; $R_4 = O$ ; $R_6 = CH = CH_2$).

D'autres dérivés de labdane répondant à la formule précitée ont été préparés par synthèse. On se reportera en particulier aux documents suivants : FR-2 372 822 ; S.V.BHAT et al., J. Chem. Soc., Perkin Trans. 1982, volume 1, page 767 ; SV.BHAT et al., J. Med. Chem. 1983, volume 26, pages 486-492 ; EP-A-252 482 ; EP-A-217 372 ; E. SEGUIN et al., Planta Medica, 1988, volume 54 n° 1, pages 4-6.

Ces documents révèlent en outre que ces composés présentent certaines propriétés pharmacologiques et en particulier une activité hypotensive et calmante du système nerveux central.

Par ailleurs, le document EP-A-0 120 165 décrit l'utilisation locale d'associations comprenant un β-stimulant et un $\alpha_2$-inhibiteur, pour le traitement des charges graisseuses. Ce document cite la forskoline à titre d'exemple de β-stimulant.

La présente invention a pour but de résoudre le nouveau problème technique consistant en la préparation d'une nouvelle formulation d'une composition cosmétique ou pharmaceutique, notamment dermatologique, comportant à titre d'ingrédient actif un labdane ou un extrait de plante en contenant et permettant de potentialiser l'activité de cet ingrédient, en particulier vis-à-vis de la stimulation de la repousse des cheveux, ou le ralentissement de leur chute.

Il a été en effet découvert de manière totalement inattendue, et ceci constitue la base de la présente invention, que les labdanes ou les extraits végétaux en contenant présentaient une activité stimulante de la repousse des cheveux et ralentissant leur chute. Il a en outre été découvert que cette activité, qui reste faible pour certains de ces composés, peut être très fortement potentialisée par leur incorporation dans des phases lamellaires lipidiques hydratées ou dans des liposomes.

Ainsi, selon un premier aspect, la présente invention concerne une composition a base de phases lamellaires lipidiques hydratées ou de liposomes, caractérisée en ce que lesdites phases lamellaires lipidiques hydratées ou lesdits liposomes contiennent au moins en partie un dérivé de labdane,de la formule I ci-après, et/ou un extrait de plante le contenant:

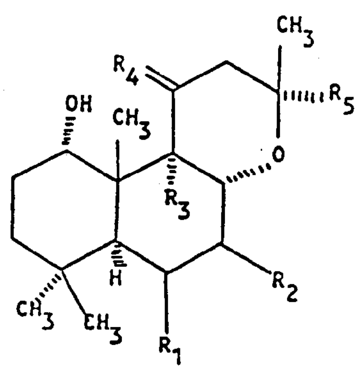

(I)

dans laquelle :

- $R_1$ et $R_2$ sont identiques ou différents et représentent chacun un groupe hydroxy ou un groupe -O-CO-$R_6$- dans lequel $R_6$ représente un radical alcoyle ayant de 1 à 7 atomes de carbone, alcoxy ayant de 1 à 7 atomes de carbone ou alcényle ayant de 1 à 7 atomes de carbone, éventuellement substitué par un ou plusieurs groupes hydroxy ou

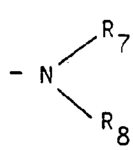

dans lequel $R_7$ et $R_8$ représentent chacun un atome d'hydrogène, un radical alcoyle ayant de 1 à 4 atomes de carbone tel que méthyle ou éthyle ou bien représentent ensemble et avec l'atome d'azote un radical hétérocyclique tel que pipéridino, morpholino, N'-méthyl-pipérazino ;
- $R_3$ représente un atome d'hydrogène ou un groupe hydroxy ;
- $R_4$ représente un atome d'oxygène ou l'ensemble

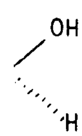

- $R_6$ représente un radical éthyle ou vinyle.

Selon une caractéristique particulière, le dérivé de labdane est un composé de formule (I) précitée dans

laquelle :

- $R_1$ représente le groupe hydroxy ;
- $R_2$ représente un groupe hydroxy ou un groupe -O-CO-$R_6$ dans lequel $R_6$ représente un radical alcoyle ayant de 1 à 4 atomes de carbone, un radical alcoxy ayant de 1 à 4 atomes de carbone ou un radical alcényle ayant 1 à 4 atomes de carbone, éventuellement substitué par un ou deux groupes hydroxy ou bien, en une extrémité de chaîne, par un groupe

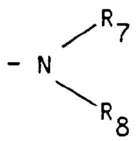

dans lequel $R_7$ et $R_8$ représentent chacun un radical méthyle ou éthyle ou bien représentent ensemble et avec l'atome d'azote, un radical hétérocyclique tel que pipéridino, morpholino, N'-méthylpipérazino ;
- $R_3$ représente le groupe hydroxy ;
- $R_4$ représente un atome d'oxygène ;
- $R_8$ représente le radical vinyle.

Les radicaux alcoyl, représentés par $R_8$, $R_7$ et $R_8$ dans les formules précitées peuvent être à chaîne droite, ramifiée ou cyclique.

Un radical alcoyl $C_1$-$C_7$ est par exemple un radical méthyle, éthyle, propyle, isopropyle, cyclopropyle, isobutyle, butyle, pentyle, néopentyle, de préférence méthyle, éthyle, ou propyle.

D'autre part, les radicaux alcoxy et alcényles représentés par $R_6$ dans les formules précitées peuvent également être à chaîne droite, ramifiée ou cyclique.

Un groupe alcoxy en $C_1$-$C_7$ est par exemple un groupe méthoxy, éthoxy, propoxy, isopropoxy, cyclopropoxy, butoxy, pentoxy, de préférence méthoxy, éthoxy ou propoxy.

Un groupe alcényle en $C_1$-$C_7$ est par exemple un groupe vinyle, allyle, isopropényle, butényle, pentényle, cyclohexényle.

Avantageusement, ce dérivé labdane est choisi parmi les composés suivants :

n° 1 : forskoline ,
n° 2 : 7-O-déacétyl-forskoline
n° 3 : coléforsine
n° 4 : 7-O-déacétyl-7-β-O-propanoyl-forskoline
n° 5 : 7-O-déacétyl-7-β-O-(éthoxycarbonyl)-forskoline
n° 6 : 7-O-déacétyl-7-β-O-(propoxycarbonyl)-forskoline
n° 7 : 7-O-déacétyl-7-β-O-(2,3-dihydroxy-propanoyl)-forskoline
n° 8 : 7-O-déacétyl-7-β-O-(3-méthyl-2-buténoyl)-forskoline
n° 9 : 7,0-déacétyl-7-β-O-[4-(N'-méthyl-pipérazino)-butanoyl]forskoline
n° 10 : 7-O-déacétyl-7-β-O-(4-morpholino-butanoyl)-forskoline
n° 11 : 6-β-O-[3-pipéridino-propanoyl]-forskoline
n° 12 : 6-β-O-(pipéridino-acétyl)-forskoline

Ces dérivés de labdane sont des composés de formule (I) précitée dans laquelle $R_3$ = OH, $R_4$ = O, $R_5$ = CH = $CH_3$ et $R_1$ et $R_2$ ont les significations figurant au tableau I ci-après :

Tableau I

| n° | $R_1$ | $R_2$ |
|---|---|---|
| 1 | OH | OCOMe |
| 2 | OH | OH |
| 3 | OCOMe | OH |
| 4 | OH | OCOEt |
| 5 | OH | OCOOEt |
| 6 | OH | OCOOPr |
| 7 | OH | $OCO-CH(OH)-CH_2OH$ |
| 8 | OH | $O-CO-CH=C(CH_3)_2$ |
| 9 | OH | 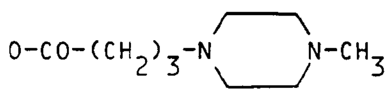 |
| 10 | OH | 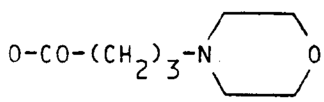 |
| 11 | 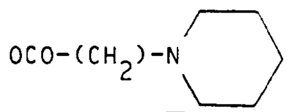 | OCOMe |
| 12 | 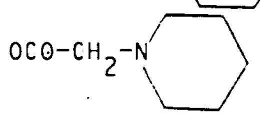 | OCOMe |

Selon un autre mode de réalisation de l'invention, on utilise, pour la préparation d'une telle composition, un extrait contenant un dérivé de labdane tel que défini précédemment, qui est par exemple un extrait de Coleus, en particulier un extrait de Coleus forskolii, de préférence un extrait de racines de la plante Coleus.

Avantageusement, il s'agit d'un extrait organique de Coleus, notamment de racines de Coleus, de préférence obtenu par un procédé comprenant au moins une étape d'extraction avec un solvant choisi parmi le groupe constitué par l'acétate d'éthyle, le méthanol, l'éthanol et le dichlorométhane.

Toutefois, on peut utiliser comme solvant des solvants organiques, tels que les hydrocarbures aromatiques, des dialcoyliques, hydrocarbures aliphatiques et aromatiques halogénés, des éthers dialcoyliques, des dialcoylcétones, des alcanols, des acides carboxyliques et leurs esters ; ou d'autres solvants, comme par exemple le diméthylformamide, le dioxanne, le tétrahydrofuranne et le diméthylsulfoxyde.

Parmi les solvants précités, des solvants préférés sont le benzène, le toluène ou le xylène, le chlorure de méthylène, le chloroforme, l'acétate d'éthyle, le méthanol ou l'éthanol.

Le rapport de la matière végétale à l'agent d'extraction n'est pas critique et généralement sera compris entre 1:5 et 1:20 parties en poids, et de préférence de 1:10 parties en poids environ.

L'extraction s'effectue à des températures comprises entre la température ambiante et le point d'ébullition du solvant utilisé pour l'extraction.

Une technique particulièrement avantageuse d'extraction est la technique dite d'extraction au Soxhlet. Il peut être avantageux, et dans certains cas nécessaire d'évaporer le solvant, par exemple par lyophilisation, et de reprendre les extraits bruts en vue d'une purification.

Dans le cadre de la présente invention, l'extraction alcoolique est particulièrement intéressante, notamment en fin de procédure d'obtention de l'extrait en raison du caractère habituellement peu toxique des alcools. Un alcool particulièrement avantageux est l'éthanol.

Un autre solvant, particulièrement intéressant est l'acétate d'éthyle, parcequ'il fournit un extrait riche en dérivé de labd-14-ène.

Des variantes particulières de procédé sont également décrites dans la littérature, en particulier dans les

documents rappelés dans la partie introductive de la présente description.

Le terme "lipidique" dans l'expression "phases lamellaires lipidiques" couvre toutes les substances comprenant une chaîne carbonée dite grasse, comportant généralement plus de 5 atomes de carbone, cette substance étant habituellement dénommée "lipide".

Selon l'invention, on utilise à titre de lipide, pour former les phases lamellaires lipidiques, ou les liposomes, des lipides amphiphiles, c'est-à-dire constitués de molécules possédant un groupe hydrophile indifféremment ionique ou non-ionique et un groupe lipophile, ces lipides amphiphiles étant susceptibles de former des phases lamellaires lipidiques ou des liposomes, en présence d'une phase aqueuse, selon la quantité d'eau dans le mélange.

En particulier, parmi ces lipides, on peut citer : les phospholipides, les phosphoaminolipides, les glycolipides, les alcools gras polyoxyéthylénés, les esters de polyols éventuellement polyoxyéthylénés. De telles substances sont par exemple constituées par une lécithine d'oeuf ou de soja, une phosphatidylsérine, une sphyngomyéline, un cérébroside ou un stéarate de polyglycérol oxyéthyléné.

De préférence, selon l'invention, on utilise un mélange lipidique constitué d'au moins un lipide amphiphile et d'au moins un lipide hydrophobe tel que stérol, comme le cholestérol ou le β-sitostérol. La quantité, exprimée en moles, de lipide hydrophobe ne doit généralement pas être supérieure à la quantité de lipide amphiphile, et de préférence elle ne doit pas être supérieure à 0,5 fois cette quantité.

L'incorporation dans des phases lamellaires lipidiques hydratées ou dans des liposomes, des composés ou des extraits contenant ces composés, utilisée conformément à la présente invention, peut être réalisée selon des techniques de préparation connues, décrites par exemple dans le document EP-B 1-0087993, et éventuellement en combinaison avec le document EP-B 1-0107559.

Selon un second aspect, la présente invention concerne une composition cosmétique ou pharmaceutique, notamment dermatologique, destinée notamment à favoriser la repousse des cheveux ou à ralentir leur chute ou à favoriser la pigmentation de l'épiderme ou prévenir l'apparition ou traiter les cheveux gris, caractérisée en ce qu'elle comprend, à titre d'ingrédient actif, au moins un dérivé de labdane, de la formule I précitée, et/ou un extrait de plantes en contenant, au moins en partie incorporé dans des phases lamellaires lipidiques hydratées ou dans des liposomes.

D'une façon générale, les compositions cosmétiques ou pharmaceutiques, notamment dermatologiques, conformes à la présente invention seront réalisées à partir des compositions à base de phases lamellaires lipidiques hydratées ou de liposomes décrites précédemment.

La concentration de dérivé de labdane ou d'extrait le contenant, incorporé au moins en partie dans des phases lamellaires lipidiques hydratées ou dans des liposomes, sera de préférence comprise entre 0,0001 % et 1% en poids, de préférence encore entre 0,01 % et 0,1 % en poids, par rapport au poids total de la composition cosmétique ou pharmaceutique.

Ces proportions s'entendent en poids sec lorsqu'il s'agit des extraits végétaux.

Selon une variante de réalisation, une composition cosmétique ou pharmaceutique, notamment dermatologique selon l'invention, comprend en outre au moins une autre substance active, à une concentration efficace, choisie parmi les xanthines, les vitamines, en particulier les vitamines B, la tyrosine ou ses dérivés comme par exemple le tyrosinate de glucose, la quinine ou ses dérivés, les rubéfiants tels que le nicotinate de méthyle, un surnageant de culture de fibroblastes de papilles, tel que défini dans le document EP-A-272 920, des hydrolysats de kératine, des oligo-éléments tels que zinc, sélénium, cuivre, des inhibiteurs de 5 α-réductasetels que : progestérone, cyprotérone acétate, Minoxidil, acide azélaïque et ses dérivés, un 4-méthyl-4-azastéroïde, en particulier la 17-β-N,N-diéthylcarbamoyl-4-méthyl-4-aza-5-α-androstan-3-one, ou encore un extrait de Serenoa repens, ladite substance active étant éventuellement incorporée au moins en partie dans lesdites phases lamellaires lipidiques hydratées ou les liposomes.

Les compositions cosmétiques, conformes à la présente invention, peuvent être appliquées par voie topique pour favoriser la repousse des cheveux, ralentir leur chute, ou encore pour favoriser la pigmentation de l'épiderme ou prévenir l'apparition ou traiter les cheveux gris, en particulier dans des compositions se présentant sous forme de crèmes, de gels ou de lotions destinés à l'application topique sur les cheveux.

Sous cet aspect, la présente invention fournit également un procédé de traitement des cheveux destiné notamment à favoriser leur repousse et à ralentir leur chute ou à favoriser la pigmentation de l'épiderme ou prévenir l'apparition ou traiter les cheveux gris, caractérisé en ce qu'il comprend l'application, en une quantité efficace, pour réaliser l'effet de repousse des cheveux, ou pour ralentir leur chute, favoriser la pigmentation de l'épiderme ou prévenir l'apparition ou traiter les cheveux gris, d'au moins une composition à base de phases lamellaires lipidiques hydratées ou de liposomes, telle que définie précédemment, éventuellement en association avec un excipient, véhicule ou support pharmaceutiquement ou cosmétiquement acceptable.

Il est à noter que par l'expression "au moins en partie incorporé dans des phases lamellaires lipidiques hydratées ou dans des liposomes" on entend dans la présente description et dans les revendications que le

dérivé de labdane ou l'extrait de plantes en contenant est combiné à des phases lamellaires lipidiques hydratées ou à des liposomes, quelle que soit la forme de cette combinaison.

Cependant, il est clair qu'une combinaison préférée selon l'invention réside dans l'incorporation, ou même l'encapsulation dans les phases lamellaires lipidiques hydratées ou dans les liposomes. Mais il n'est pas nécessaire que la totalité du principe actif soit incorporée ou encapsulée pour obtenir l'effet recherché.

Selon un autre aspect, l'invention fournit encore un ,procédé de fabrication d'une composition cosmétique ou pharmaceutique, notamment dermatologique, destinée à favoriser la repousse des cheveux ou a retarder leur chute, favoriser la pigmentation de l'épiderme ou prévenir l'apparition ou traiter les cheveux gris, caractérisé en ce qu'il comprend en premier lieu l'incorporation d'au moins un labdane et/ou un extrait végétal en contenait, au moins en partie dans des phases lamellaires lipidiques hydratées ou dans des liposomes, puis leur mélange dans un excipient, véhicule ou support pharmaceutiquement ou cosmétiquement acceptable.

Sous un dernier aspect, l'invention concerne l'utilisation d'une composition à base de phases lamellaires lipidiques hydratées ou de liposomes telle que définie précédemment pour la préparation d'une composition cosmétique ou pharmaceutique, notamment dermatologique, destinée au traitement des cheveux, notamment à favoriser la repousse des cheveux ou à ralentir leur chute et à prévenir l'apparition ou à traiter les cheveux gris.

D'autres buts, caractéristiques et avantages de l'invention apparaîtront plus clairement à la lecture de la description explicative qui va suivre, faite en référence à plusieurs exemples donnés uniquement à titre d'illustration et qui ne sauraient par conséquent d'aucune façon limiter la portée de l'invention.

Dans les exemples, les pourcentages sont exprimés en poids, sauf indication contraire.

Lorsqu'il s'agit d'extraits végétaux, les poids indiqués sont des poids secs, sauf indication contraire.

## Exemple 1

Préparation d'une suspension de liposomes contenant un dérivé labd-14-ène

Dans 25 ml d'un mélange de dichlorométhane et de méthanol en proportion volumétrique 4:1, on dissout 0,398 g de lécithine de soja et 0,002 g de forskoline (1$\alpha$, 6$\beta$, 9$\alpha$-trihydroxy-7$\beta$-acétoxy-8,13-époxy-labd-14-en-11-one).

La solution est évaporée au ballon rotatif sous pression réduite à 45°C environ.

Le film lipidique obtenu est repris sous agitation avec environ 19,6 ml de solution aqueuse constituée par un tampon phosphate PBS.

On obtient une suspension de vésicules lipidiques ou liposomes qui est ensuite traitée aux ultrasons pendant 15 minutes à 4°C avec une puissance de 150 W.

On obtient ainsi une suspension de liposomes de taille sensiblement homogène, de l'ordre de 139 nm. Cette suspension contient environ 0,01 % de forskoline, incorporée dans la phase lipidique des liposomes.

Si on le souhaite, cette suspension peut être gélifiée, par exemple en la mélangeant à un volume équivalent d'un gel de Carbopol 940® préparé de façon classique.

## Exemple 2

Obtention d'un extrait de Coleus à partir de Coleus forskolii

On extrait 60 g de racines séchées et broyées de Coleus forskolii au Soxhlet avec 600 ml de solvant. L'extraction se produit à température d'ébullition du solvant avec un solvant toujours renouvelé.

Le rendement moyen d'extraction est le suivant, exprimé en poids sec :

a) Mélange éthanol/eau (80/20 v/v) : environ 9,2 à 9,5 g (extrait 2A)

b) Acétate d'éthyle : environ 1,5 g (extrait 2B)

c) Dichlorométhane : environ 4 g (extrait 2C)

## Exemple 3

Incorporation d'un extrait de Coleus forskolii dans des phases lamellaires lipidiques hydratées ou dans des liposomes

Un extrait de Coleus forskolii obtenu conformément à l'exemple 2 est incorporé dans des phases lamellaires lipidiques hydratées ou dans des liposomes selon la technique de préparation décrite dans l'exemple 1.

La préparation des liposomes est par exemple la suivante :

On pèse :

- lécithine de soja : 0,9 g
- β-sitostérol : 0,1 g
- extrait de Coleus lyophilisé (extrait 2B) : 0,025 g

Ces constituants sont dissous dans un mélange constitué de 100 ml de dichlorométhane et 25 ml de méthanol.

On évapore le mélange ainsi obtenu dans un ballon rotatif à température de 45°C sous pression réduite.

Le film lipidique ainsi obtenu est alors repris et dispersé sous agitation dans de l'eau distillée qsp 50 g, à température ambiante pendant 2 heures.

La suspension de vésicules lipidiques obtenue est ensuite homogénéisée par un traitement aux ultrasons pendant 30 minutes à 4°C, sous puissance de 150 W.

La taille moyenne des liposomes ainsi obtenus est d'environ 212 nm.

On gélifie ensuite cette suspension en la mélangeant à 50 g de gel de Carbopol 940® à 1,25% préparé séparément de façon classique. On obtient ainsi 100 g environ d'une suspension gélifiée de liposomes encapsulant l'extrait de Coleus forskolii, dont la concentration est environ 0,025 % par rapport au poids total de la suspension.

**Exemples 4 à 9**

En suivant le processus expérimental décrit à l'exemple 1, on a préparé des suspensions de liposomes à partir des compositions suivantes :

**Exemple 4**

- lécithine de soja : 2 g
- β-sitostérol : 0,05 g
- extrait de Coleus (extrait 2B) : 0,01 g
- eau distillée qsp : 50 g

**Exemple 5**

- lécithine d'oeuf : 1,8 g
- cholestérol : 0,1 g
- extrait de Coleus (extrait 2A) : 0,02 g
- eau distillée qsp : 50 g

**Exemple 6**

- lécithine de soja : 2 g
- extrait de Coleus (extrait 2C) : 0,035 g
- eau distillée qsp : 50 g

**Exemple 7**

- lécithine de soja : 2 g
- β-sitostérol : 0,1 g
- extrait de Coleus (extrait 2B) : 0,03 g
- eau distillée qsp : 50 g

**Exemple 8**

- lécithine de soja : 2 g
- progestérone : 0,001 g
- extrait de Coleus (extrait 2A) : 0,0275 g
- eau distillée qsp : 45 g

**Exemple 9**

- lécithine de soja : 2 g
- extrait de Coleus (extrait 2C) : 0,025 g
- extrait de Serenoa repens : 0,01 g
- eau distillée qsp : 50 g

**Exemple 10**

Mise en évidence d'une activité anti-chute sur la croissance des cheveux

On met en évidence l'activité anti-chute sur la croissance des cheveux en étudiant l'activité des produits selon l'invention sur le cycle pilaire de rats Sprague Dawley âgés de 23 jours. Les rats sont rasés, au niveau de la partie inférieure du dos, le 24e jour.

On applique ensuite (à partir du 25e jour) et jusqu'au 65e jour, 6 jours sur 7, les produits à tester, à une dose allant de 0,5 ml à 2 ml en fonction de l'évolution pondérale des aminaux.

A intervalles de temps sensiblement réguliers (environ tous les 3 jours) on prélève, à l'aide d'une pince à épiler, une petite touffe d'au moins 10 poils sur le côté gauche de l'animal au niveau du flanc.

On compte alors le pourcentage de poils en phase anagène (phase de croissance), en fonction du temps. L'identification des poils en phase anagène est effectuée par l'observation microscopique de l'extrémité inférieure du poil. La base des poils en phase anagène est fine, car la partie vivante est restée dans le derme, tandis que la base des poils en phase télogène (phase de repos) est en forme de massue. Les poils en phase catagène (phase intermédiaire de régression) sont toujours très peu nombreux.

L'étude est effectuée sur 30 rats répartis en 3 lots de 10 animaux. Le premier lot reçoit le produit de l'exemple 3. Le second reçoit une solution alcoolique du même extrait de Coleus à la même concentration de 0,025%. Le troisième lot est le lot témoin ne recevant aucun produit.

Les résultats de cette étude trichocinétique sont donnés sur la figure unique annexée.

Sur cette figure, on a représenté en ordonnées le pourcentage de poils en phase anagène, et en abscisses le nombre de jours.

La courbe joignant les triangles dont la pointe est dirigée vers le haut correspond aux résultats obtenus avec des liposomes incorporant 0,025 % d'extrait de Coleus, et préparés selon l'exemple 3.

La courbe joignant les triangles dont la pointe est dirigée vers le bas correspond aux résultats obtenus avec le lot témoin.

La courbe joignant des cercles correspond aux résultats obtenus avec un extrait de Coleus dans l'alcool, à l'état libre, à la même teneur de 0,025 % en poids, par rapport au poids total de la composition.

On observe que le nombre de poils en phase anagène croît beaucoup plus rapidement dans le lot recevant l'extrait de Coleus en liposomes selon l'invention, que dans celui recevant la solution alcoolique d'extrait de Coleus (respectivement 65% et 25% au jour 37). On observe également que la phase anagène se prolonge plus longtemps.

Par contre, si on compare la courbe correspondant à la solution alcoolique d'extrait de Coleus avec la courbe témoin, on n'observe pas de différence vraiment significative.

Ainsi, il est clair que les extraits de Coleus incorporés dans les liposomes, selon l'invention, par l'allongement de la durée de la phase anagène, ralentissent très nettement la chute des cheveux et favorisent leur repousse.

**Exemple 11**

Mesure de l'activité pigmentante d'un extrait de Coleus forskohlii incorporédans des liposomes sur la pigmentation cutanée chez le cobaye

Protocole :

L'étude est menée sur un lot de 10 cobayes tricolores.

Avant et pendant l'expérimentation, les flancs droit et gauche des cobayes sont soigneusement rasés, chaque jour durant les 5 premiers jours (période d'exposition aux U.V.), puis tous les 2 jours jusqu'à la fin de l'étude.

Pour chaque animal, on détermine sur chaque flanc des taches comparablement pigmentées, d'aspect le plus souvent marron clair. Sur l'un des deux flancs tirés au hasard, on applique 10 min avant l'exposition aux rayons ultraviolets 0,5 g environ de produit à tester ou de produit témoin, selon le lot, l'autre flanc étant exposé

"nu" à titre de contrôle.

L'application des produits à tester est effectuée dès le premier jour d'exposition, jusqu'au sacrifice de l'animal.

L'exposition aux rayonnements ultraviolets est effectuée au moyen d'un simulateur solaire délivrant 86% d'U.V.A. et 14% d'U.V.B. pendant les 5 premiers jours de l'expérimentation, à raison de 5 min le premier jour, 10 min le deuxième jour, 15 min le troisième jour et 20 min les quatrième et cinquième jours.

Les animaux sont sacrifiés 12 jours après la dernière exposition, et l'on procède à une biopsie cutanée.

On prélève ainsi un fragment de peau sur le flanc non traité mais exposé, ainsi que sur l'autre flanc traité et exposé.

On effectue ensuite un examen histologique des fragments cutanés.

Cet examen comporte : d'une part, l'étude de la mélanogénèse par la méthode Argentaffine de Fontana sur des coupes de 4 >m (Techniques d'histologie, Professeur Chevreau, Ed. Maloine, 1977, page 157), d'autre part, l'appréciation de l'épaisseur de l'épiderme sur des coupes de 4 >m colorées selon la méthode trichomique de Masson.

L'étude de l'épaisseur de l'épiderme et de l'intensité de la mélanogénèse permet d'apprécier un effet bronzant ou plus exactement l'activation du processus de mélanogénèse.

Pour étudier la mélanogénèse, on examine deux zones tirées au hasard d'une tache pigmentée, dans lesquelles on repère 25 cellules malpigiennes et on compte parmi celles-ci les mélanocytes "activés", c'est-à-dire contenant de la mélanine en amas. On exprime alors l'activation du processus de la mélanogénèse en pourcentage de cellules activées à partir de la moyenne de ces deux valeurs.

Sur ces mêmes zones, on examine la quantité de mélanine dans les autres couches de l'épiderme et on apprécie globalement cette quantité suivant une échelle à cinq valeurs variant de 0 à 4 selon que la quantité de mélanine formée est nulle, faible, moyenne, importante ou très importante.

On a regroupé au tableau I les résultats de l'étude histologique donnant le pourcentage d'activation et permettant d'apprécier les variations de la quantité de mélanine formée (valeurs moyennes selon l'échelle définie ci-dessus), de l'épaisseur de l'épiderme (exprimée en >m).

Le produit testé est constitué par un extrait de Coleus à l'acétate d'éthyle selon l'exemple 2b, incorporé en liposomes selon l'exemple 3, mais titrant 0,0284% d'extrait de Coleus au lieu de 0,025%.

TABLEAU I

| | Flanc contrôle | Flanc traité |
|---|---|---|
| % Activation | 33,40 | 85,30 |
| Quantité de mélanine | 0,16 | 2,39 |
| Epaississement de l'épiderme en >m | 8,32 | 14,10 |

On peut constater à partir du tableau I que l'extrait de Coleus incorporé en liposomes selon l'invention est actif sur la mélanogénèse. La lecture des coupes histologiques des flancs traités montre un taux très important de mélanocytes activés présentant des formes rhizomiques, et une migration importante des grains de mélanines dans l'épiderme.

Ces résultats confirment donc de manière claire l'activité des extraits de Coleus incorporés en liposomes selon l'invention sur l'activation des mélanocytes.

On donnera ci-après divers exemples de formulation de compositions cosmétiques ou pharmaceutiques, favorisant la repousse des cheveux et/ou ralentissant leur chute et/ou prévenant ou traitant l'apparition des cheveux gris. Les constituants hydrosolubles peuvent être avantageusement dissous dans la phase aqueuse dans laquelle est dispersée la poudre lipidique, comme indiqué à l'exemple 3. Ainsi, ces constituants peuvent être incorporés, au moins en partie, dans les liposomes.

**Exemple 12**

Lotion freinant la chute des cheveux

Suspension de liposomes selon l'exemple 4 50 g

- Carbopol 940® 0,03 g
- eau distillée qsp 100 ml

On applique cette lotion sur le cuir chevelu 2 fois par jour pendant 6 mois.

**Exemple 13**

Lotion anti-chute des cheveux

Suspension de liposomes selon l'exemple 5 50 g

- Carbopol 940® 0,03 g
- panthénol 0,1 g
- hydrolysat kératine 0,2 g
- parfum hydrosoluble 0,1 g
- eau distillée qsp 100 ml

On applique cette lotion sur le cuir chevelu 2 fois par jour pendant 6 mois.

**Exemple 14**

Lotion stimulante du bulbe pileux

Suspension de liposomes selon l'exemple 6 50 g

- Carbopol 940® 0,04 g
- Phytantriol® 0,1 g
- conservateurs 0,05 g
- complexe protéine-zinc 0,1 g
- eau qsp 100 ml

On applique cette lotion sur le cuir chevelu, sur les zones de chute, 5 jours sur 7, par cure de 6 mois. Cette application est suivie d'un léger massage.

**Exemple 15**

Lotion favorisant la repousse des cheveux et prévenant l'apparition des cheveux gris

Suspension de liposomes selon l'exemple 7 50g

- Carbopol 940® 0,05
- tyrosinate de glucose 0,05
- complexe oligoéléments 0,1
- théophylline 0,01
- conservateurs 0,05
- eau distillée qsp 100 ml

On applique cette lotion le soir sur le cuir chevelu au niveau des zones grisonnantes et des zones de chute, par cure de 4 mois.

**Exemple 16**

Gel coiffant anti-chute

Suspension de liposomes selon l'exemple 8 45 g

- parfum 0,1 g
- gel Carbopol 940® à 1,50 % 50 g
- eau distillée qsp 100 g

Appliquer ce gel, de préférence après le shampooing, 2 fois par semaine, par cure de 2 mois.

**Exemple 17**

Gel anti chute contre l'alopécie séborrhéique

Suspension de liposomes selon l'exemple 9 50 g
- parfum 0,1
- complexe proteine-zinc 0,05
- gel carbopol à 1,50 % 45 g
- eau qsp 100 g

On applique ce gel, de préférence chaque soir sur les zones de chute, par cure de 4 mois.

**Exemple 18**

Lotion freinant la chute des cheveux

Suspension de liposomes selon l'exemple 1 20 g
- Carbopol 940® 0,03 g
- eau distillée qsp 100 ml

**Exemple 19**

Gel bronzant

Suspension de liposomes (non gélifiée) selon l'exemple 3 50 g
- Carbopol 940® 1,25 g
- filtre solaire hydrosoluble 6 g
- eau distillée qsp 100 g

**Exemple 20**

Lotion favorisant la repousse des cheveux

Suspension de liposomes selon l'exemple 7 mais contenant dans la phase aqueuse de l'hydrolysat de kératine humaine à concentration de 5%... 50 g
- Carbopol 940® 0,05 g
- complexe oligo-éléments 0,1 g
- conservateur 0,05 g
- eau distillée qsp 100 ml

La suspension de liposomes selon l'exemple 7 contenant dans la phase aqueuse de l'hydrolysat de kératine humaine à 5% est préparée conformément à l'exemple 3 sauf que le film lipidique est repris dans une solution aqueuse contenant 5% d'hydrolysat de kératine humaine.

On applique cette solution le soir sur le cuir chevelu au niveau des zones de chute, par cure de 4 mois.

## Revendications

**Revendications pour les Etats contractants suivants : AT, BE, CH, LI, DE, DK, GB, IT, LU, NL, SE**

1. Composition à base de phases lamellaires lipidiques hydratées ou de liposomes, caractérisée en ce que lesdites phases lamellaires lipidiques hydratées ou lesdits liposomes contiennent au moins en partie un dérivé de labdane, de la formule I ci-après, et/ou un extrait de plante le contenant :

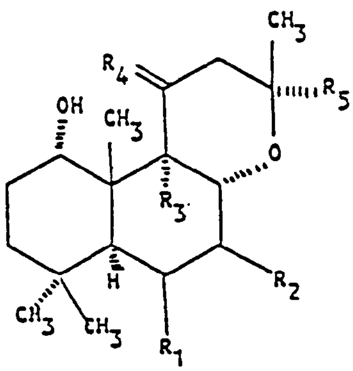

(I)

dans laquelle :

- $R_1$ et $R_2$ sont identiques ou différents et représentent chacun un groupe hydroxy ou un groupe -O-CO-$R_6$ dans lequel $R_6$ représente un radical alcoyle ayant de 1 à 7 atomes de carbone, alcoxy ayant de 1 à 7 atomes de carbone ou alcényle ayant de 1 à 7 atomes de carbone, éventuellement substitué par un ou plusieurs groupes hydroxy ou

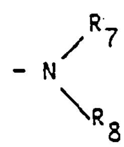

  dans lequel $R_7$ et $R_8$ représentent chacun un atome d'hydrogène, un radical alcoyle ayant de 1 à 4 atomes de carbone tel que éthyle ou bien représentent ensemble et avec l'atome d'azote un radical hétérocyclique tel que pipéridino, morpholino, N'-méthyl-pipérazino ;
- $R_3$ représente un atome d'hydrogène ou un groupe hydroxy ;
- $R_4$ représente un atome d'oxygène ou l'ensemble

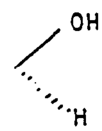

- $R_8$ représente un radical éthyle ou vinyle.

**2.** Composition selon la revendication 1, caractérisée en ce que le dérivé de labdane précité est un composé de formule (I) dans laquelle :

- $R_1$ représente le groupe hydroxy ;
- $R_2$ représente un groupe hydroxy ou un groupe -O-CO-$R_8$ dans lequel $R_8$ représente un radical alcoyle ayant de 1 à 4 atomes de carbone, un radical alcoxy ayant de 1 à 4 atomes de carbone ou un radical alcényle ayant de 1 à 4 atomes de carbone, éventuellement substitué par un ou deux groupes hydroxy ou bien, en (une extrémité de chaîne par un groupe

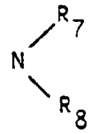

  dans lequel $R_7$ et $R_8$ représentent chacun un radical méthyle ou éthyle ou bien représentent ensemble et avec l'atome d'azote un radical hétérocyclique tel que pipéridino, morpholino, N'-méthylpipérazino ;
- $R_3$ représente le groupe hydroxy,

- $R_4$ représente un atome d'oxygène,
- $R_5$ représente le radical vinyle.

3. Composition selon la revendication 2, caractérisée en ce que le dérivé de labdane précité est l'un des composés suivants :
   - forskoline
   - 7-O-déacétyl-forskoline
   - coléforsine
   - 7-O-déacétyl-7-β-O-propanoyl-forskoline
   - 7-O-déacétyl-7-β-O-(éthoxycarbonyl)-forskoline
   - 7-O-déacétyl-7-β-O-(propoxycarbonyl)-forskoline
   - 7-O-déacétyl-7-β-O-(2,3-dihydroxy-propanoyl)-forskoline
   - 7-O-déacétyl-7-β-O-(3-méthyl-2-buténoyl)-forskoline
   - 7,0-déacétyl-7-β-O-[4-(N'-méthyl-pipérazino)-butanoyl]-forskoline
   - 7-O-déacétyl-7-β-O-(4-morpholino-butanoyl)-forskoline
   - 6-β-O-[3-pipéridino-propanoyl]-forskoline
   - 6-β-O-(pipéridino-acétyl)-forskoline

4. Composition selon la revendication 1 , caractérisée en ce que l'extrait contenant un dérivé de labdane précité est un extrait de Coleus, en particulier un extrait de Coleus forskohlii, de préférence un extrait de racines de la plante Coleus.

5. Composition selon la revendication 4 , caractérisée en ce que l'extrait de Coleus précité est un extrait organique de Coleus, notamment de racines de Coleus, de préférence obtenu par un procédé comprenant au moins une étape d'extraction avec l'acétate d'éthyle.

6. Composition cosmétique ou pharmaceutique, notamment dermatologique, destinée notamment à favoriser la repousse des cheveux ou à ralentir leur chute, ou à favoriser la pigmentation de l'épiderme ou prévenir l'apparition ou traiter les cheveux gris, caractérisée en ce qu'elle comprend, à titre d'ingrédient actif, un dérivé de labdane, de la formule I ci-après, et/ou un extrait de plante en contenant, au moins en partie incorporé dans des phases lamellaires lipidiques hydratées ou dans des liposomes :

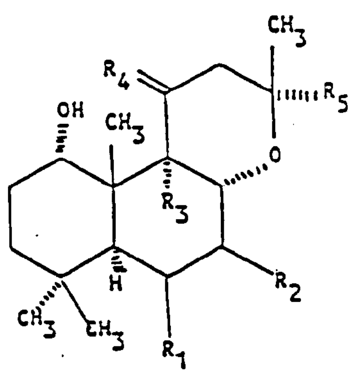

(I)

dans laquelle :

- les groupes $R_1$ et $R_2$ sont identiques ou différents et représentent chacun un groupe hydroxy ou un groupe -O-CO-$R_6$ dans lequel $R_5$ représente un radical alcoyle ayant de 1 à 7 atomes de carbone, alcoxy ayant de 1 à 7 atomes de carbone ou alcényle ayant de 1 à 7 atomes de carbone, éventuellement substitué par un ou plusieurs groupes hydroxy ou

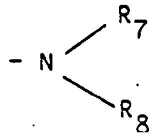

dans lequel $R_7$ et $R_8$ représentent chacun un atome d'hydrogène, un radical alcoyle ayant 1 à 4 atomes de carbone tel que méthyle ou éthyle ou bien représentent ensemble et avec l'atome d'azote un radical hétérocyclique tel que pipéridino, morpholino, N'-méthyl-pipérazino ;

- $R_3$ représente un atome d'hydrogène ou un groupe hydroxy ;
- $R_4$ représente un atome d'oxygène ou l'ensemble

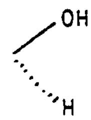

- $R_8$ représente un radical éthyle ou vinyle.

7. Composition cosmétique ou pharmaceutique, notamment dermatologique, selon la revendication 6, caractérisée en ce que le dérivé de labdane précité est un composé de formule (I) précitée dans laquelle :
   - $R_1$ représente le groupe hydroxy ;
   - $R_2$ représente un groupe hydroxy ou un groupe -O-CO-$R_8$ dans lequel $R_8$ représente un radical alcoyle ayant de 1 à 4 atomes de carbone, un radical alcoxy a yant de 1 à 4 atomes de carbone ou un radical alcényle ayant de 1 à 4 atomes de carbone, éventuellement substitué par un ou deux groupes hydroxy ou bien, en une extrémité de chaîne par un groupe

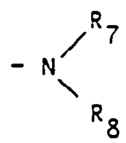

   dans lequel $R_7$ et $R_8$ représentent chacun un radical méthyle ou éthyle ou bien représentent ensemble et avec l'atome d'azote voisin, un radical hétérocyclique tel que pipéridino, morpholino, N'-méthylpipérazino ;
   - $R_3$ représente le groupe hydroxy,
   - $R_4$ représente un atome d'oxygène,
   - $R_8$ représente le radical vinyle.

8. Composition cosmétique ou pharmaceutique, notamment dermatologique, selon l'une des revendications 6 et 7, caractérisée en ce que le dérivé de labdane est l'un des composés suivants :
   - forskoline
   - 7-O-déacétyl-forskoline
   - coléforsine
   - 7-O-déacétyl-7-β-O-propanoyl-forskoline
   - 7-O-déacétyl-7-β-O-(éthoxycarbonyl)-forskoline
   - 7-O-déacétyl-7-β-O-(propoxycarbonyl)-forskoline
   - 7-O-déacétyl-7-β-O-(2,3-dihydroxy-propanoyl)-forskoline
   - 7-O-déacétyl-7-β-O-(3-méthyl-2-buténoyl)-forskoline
   - 7,0-déacétyl-7-β-O-[4-(N'-méthyl-pipérazino)-butanoylΩ-forskoline
   - 7-O-déacétyl-7-β-O-(4-morpholino-butanoyl)-forskoline
   - 6-β-O-[3-pipéridino-propanoylΩ-forskoline
   - 6-β-O-(pipéridino-acétyl)-forskoline

9. Composition cosmétique ou pharmaceutique, notamment dermatologique, selon la revendication 6, caractérisée en ce que l'extrait contenant un dérivé de labdane précité est un extrait de Coleus, en particulier un extrait de Coleus forskohlii, de préférence un extrait de racines de Coleus.

10. Composition cosmétique ou pharmaceutique, notamment dermatologique, selon la revendication 9 , caractérisée en ce que l'extrait de Coleus précité est un extrait organique de Coleus, notamment de racines ce Coleus, de préférence obtenu par un procédé comprenant au moins une étape d'extraction avec l'acétate d'éthyle.

**11.** Composition cosmétique ou pharmaceutique, notamment dermatologique, selon l'une des revendications 6 à 10, caractérisée en ce que la concentration de dérivé de labdane précité ou d'extrait le contenant, incorporé au moins en partie dans des phases lamellaires lipidiques hydratées ou dans des liposomes, est comprise entre 0,0001 % et 1% en poids, de préférence entre 0,01 % et 0,1 % en poids, par rapport au poids total de la composition.

**12.** Composition cosmétique ou pharmaceutique, notamment dermatologique, selon l'une des revendications 6 à 11, caractérisée en ce qu'elle comprend en outre au moins une autre substance active à une concentration efficace, choisie parmi les xanthines, les vitamines, en particulier les vitamines B, la tyrosine ou ses dérivés comme par exemple le tyrosinate de glucose, la quinine.ou ses dérivés, les rubéfiants tels que le nicotinate de méthyle, un surnageant de culture de fibroblastes de papilles, des hydrolysats de kératine, des oligo-éléments tels que zinc, sélénium, cuivre, des inhibiteurs de 5 $\alpha$-réductase tels que : progestérone, cyprotérone acétate, Minoxidil, acide azélaïque et ses dérivés, un 4-méthyl-4-azastéroïde, en particulier la 17-$\beta$-N,N-diéthylcarbamoyl-4-méthyl-4-aza-5-$\alpha$-androstan-3-one, ou encore un extrait de Serenoa repens, ladite substance active étant éventuellement incorporée au moins en partie dans lesdites phases lamellaires lipidiques hydratées ou les liposomes.

**13.** Utilisation d'une composition à base de phases lamellaires lipidiques hydratées ou de liposomes selon l'une des revendications 1 à 5, pour la préparation d'une composition cosmétique ou pharmaceutique, notamment dermatologique, destinée au traitement des cheveux, notamment pour favoriser la repousse des cheveux ou pour ralentir leur chute et pour prévenir l'apparition ou pour traiter les cheveux gris.

**Revendications pour l'Etat contractant suivant : ES**

**1.** Procédé de préparation d'une composition à base de phases lamellaires lipidiques hydratées ou de liposomes, caractérisé en ce qu'on incorpore dans lesdites phases lamellaires lipidiques hydratées ou lesdits liposomes, au moins en partie un dérivé de labdane, de la formule I ci-après, et/ou un extrait de plante le contenant:

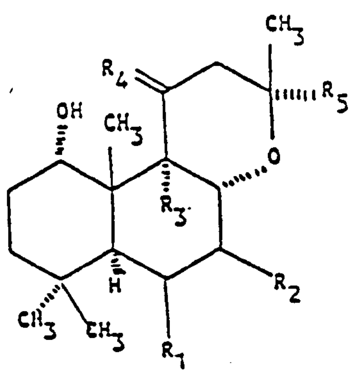

(I)

dans laquelle :

- $R_1$ et $R_2$ sont identiques ou différents et représentent chacun un groupe hydroxy ou un groupe -O-CO-$R_6$ dans lequel $R_6$ représente un radical alcoyle ayant de 1 à 7 atomes de carbone, alcoxy ayant de 1 à 7 atomes de carbone ou alcényle ayant de 1 à 7 atomes de carbone, éventuellement substitué par un ou plusieurs groupes hydroxy ou

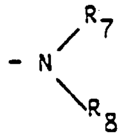

dans lequel $R_7$ et $R_8$ représentent chacun un atome d'hydrogène, un radical alcoyle ayant de 1 à 4 atomes de carbone tel que éthyle ou bien représentent ensemble et avec l'atome d'azote un radical hétérocyclique tel que pipéridino, morpholino, N'-méthyl-pipérazino ;
- $R_3$ représente un atome d'hydrogène ou un groupe hydroxy ;

- $R_4$ représente un atome d'oxygène ou l'ensemble

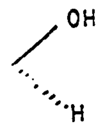

- $R_5$ représente un radical éthyle ou vinyle.

**2.** Procédé selon la revendication 1, caractérisé en ce qu'on incorpore, comme dérivé de labdane précité, un composé de formule (I) dans laquelle:
- $R_1$ représente le groupe hydroxy ;
- $R_2$ représente un groupe hydroxy ou un groupe -O-CO-$R_5$ dans lequel $R_5$ représente un radical alcoyle ayant de 1 à 4 atomes de carbone, un radical alcoxy ayant de 1 à atomes de carbone ou un radical alcényle ayant de 1 à 4 atomes de carbone, éventuellement substitué par un ou deux groupes hydroxy ou bien, en une extrémité de chaîne par un groupe

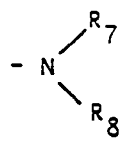

  dans lequel $R_7$ et $R_5$ représentent chacun un radical méthyle ou éthyle ou bien représentent ensemble et avec l'atome d'azote un radical hétérocyclique tel que pipéridino, morpholino, N'-méthyl-pipérazino;
- $R_3$ représente le groupe hydroxy,
- $R_4$ représente un atome d'oxygène,
- $R_5$ représente le radical vinyle.

**3.** Procédé selon la revendication 2, caractérisé en ce qu'on incorpore, comme dérivé de labdane précité, l'un des composés suivants:
- forskoline
- 7-O-déacétyl-forskoline
- coléforsine
- 7-O-déacétyl-7-β-O-propanoyl-forskoline
- 7-O-déacétyl-7-β-O-(éthoxycarbonyl)-forskoline
- 7-O-déacétyl-7-β-O-(propoxycarbonyl)-forskoline
- 7-O-déacétyl-7-β-O-(2,3-dihydroxy-propanoyl)-forskoline
- 7-O-déacétyl-7-β-O-(3-méthyl-2-buténoyl)-forskoline
- 7,0-déacétyl-7-β-O-[4-(N'-méthyl-pipérazino)-butanoyl]forskoline
- 7-O-déacétyl-7-β-O-(4-morpholino-butanoyl)-forskoline
- 6-β-O-[3-pipéridino-propanoyl]-forskoline
- 6-β-O-(pipéridino-acétyl)-forskoline

**4.** Procédé selon la revendication 1, caractérisé en ce qu'on incorpore, comme extrait contenant un dérivé de labdane précité, un extrait de Coleus, en particulier un extrait de Coleus forskohlii, de préférence un extrait de racines de la plante Coleus.

**5.** Procédé selon la revendication 4, caractérisé en ce qu'on incorpore, comme extrait de Coleus précité, un extrait organique de Coleus, notamment de racines de Coleus, de préférence obtenu par un procédé comprenant au moins une étape d'extraction avec l'acétate d'éthyle.

**6.** Procédé de préparation d'une composition cosmétique ou pharmaceutique, notamment dermatologique, destinée notamment à favoriser la repousse des cheveux ou à ralentir leur chute, ou à favoriser la pigmentation de l'épiderme ou prévenir l'apparition ou traiter les cheveux gris, caractérisé en ce qu'on utilise, à titre d'ingrédient actif, un dérivé de labdane, de la formule I ci-après, et/ou un extrait de plante en contenant, que l'on incorpore, au moins en partie dans des phases lamellaires lipidiques hydratées ou dans des liposomes:

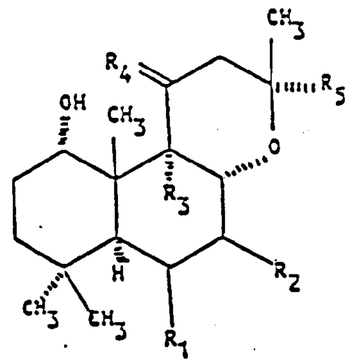

(I)

dans laquelle :

- les groupes $R_1$ et $R_2$ sont identiques ou différents et représentent chacun un groupe hydroxy ou un groupe -O-CO-$R_6$ dans lequel $R_6$ représente un radical alcoyle ayant de 1 à 7 atomes de carbone, alcoxy ayant de 1 à 7 atomes de carbone ou alcényle ayant de 1 à 7 atomes de carbone, éventuellement substitué par un ou plusieurs groupes hydroxy ou

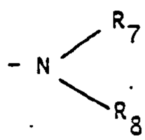

dans lequel $R_7$ et $R_6$ représentent chacun un atome d'hydrogène, un radical alcoyle ayant 1 à 4 atomes de carbone tel que méthyle ou éthyle ou bien représentent ensemble et avec l'atome d'azote un radical hétérocyclique tel que pipéridino, morpholino, N'-méthyl-pipérazino ;
- $R_3$ représente un atome d'hydrogène ou un groupe hydroxy ;
- $R_4$ représente un atome d'oxygène ou l'ensemble

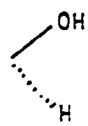

- $R_6$ représente un radical éthyle ou vinyle.

7. Procédé, selon la revendication 6, caractérisé en ce qu'on utilise comme dérivé de labdane précité, un composé de formule (I) précitée dans laquelle:
   - $R_1$ représente le groupe hydroxy ;
   - $R_2$ représente un groupe hydroxy ou un groupe -O-CO-$R_6$ dans lequel $R_6$ représente un radical alcoyle ayant de 1 à 4 atomes de carbone, un radical alcoxy a yant de 1 à 4 atomes de carbone ou un radical alcényle ayant de 1 à 4 atomes de carbone, éventuellement substitué par un ou deux groupes hydroxy ou bien, en une extrémité de chaîne par un groupe

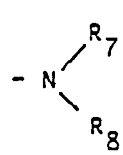

dans lequel $R_7$ et $R_6$ représentent chacun un radical méthyle ou éthyle ou bien représentent ensemble et avec l'atome d'azote voisin, un radical hétérocyclique tel que pipéridino, morpholino, N'-méthylpipérazino ;

- $R_3$ représente le groupe hydroxy,
- $R_4$ représente un atome d'oxygène,
- $R_5$ représente le radical vinyle.

**8.** Procédé selon l'une des revendications 6 et 7, caractérisé en ce qu'on utilise comme dérivé de labdane précité, l'un des composés suivants:
- forskoline
- 7-O-déacétyl-forskoline
- coléforsine
- 7-O-déacétyl-7-β-O-propanoyl-forskoline
- 7-O-déacétyl-7-β-O-(éthoxycarbonyl)-forskoline
- 7-O-déacétyl-7-β-O-(propoxycarbonyl)-forskoline
- 7-O-déacétyl-7-β-O-(2,3-dihydroxy-propanoyl)-forskoline
- 7-O-déacétyl-7-β-O-(3-méthyl-2-buténoyl)-forskoline
- 7,0-déacétyl-7-β-O-[4-(N'-méthyl-pipérazino)-butanoylΩ-forskoline
- 7-O-déacétyl-7-β-O-(4-morpholino-butanoyl)-forskoline
- 6-β-O-[3-pipéridino-propanoylΩ-forskoline
- 6-β-O-(pipéridino-acétyl)-forskoline

**9.** Procédé selon la revendication 6, caractérisé en ce qu'on utilise comme extrait contenant un dérivé de labdane précité un extrait de Coleus, en particulier un extrait de Coleus forskohlii de préférence un extrait de racines de Coleus.

**10.** Procédé selon la revendication 9, caractérisé en ce qu'on utilise comme extrait de Coleus précité, un extrait organique de Coleus, notamment de racines de Coleus, de préférence obtenu par un procédé comprenant au moins une étape d'extraction avec l'acétate d'éthyle.

**11.** Procédé selon l'une des revendications 6 à 10, caractérisé en ce qu'on utilise une concentration en dérivé de labdane précité ou d'extrait le contenant, incorporé au moins en partie dans des phases lamellaires lipidiques hydratées ou dans des liposomes, comprise entre 0,0001 % et 1% en poids, de préférence entre 0,01 % et 0,1 % en poids, par rapport au poids total de la composition.

**12.** Procédé selon l'une des revendications 6 à 11, caractérisé en ce qu'on ajoute en outre au moins-une autre substance active à une concentration efficace, choisie parmi les xanthines, les vitamines, en particulier les vitamines B, la tyrosine ou ses dérivés comme par exemple le tyrosinate de glucose, la quinine ou ses dérivés, les rubéfiants tels que le nicotinate de méthyle, un surnageant de culture de fibroblastes de papilles, des hydrolysats de kératine, des oligo-éléments tels que zinc, sélénium, cuivre, des inhibiteurs de 5 α-réductase tels que : progestérone, cyprotérone acétate, Minoxidil, acide azélaïque et ses dérivés, un 4-méthyl-4-azastéroïde, en particulier,la 17-β-N,N-diéthylcarbamoyl-4-méthyl-4-aza-5-α-androstan-3-one, ou encore un extrait de Serenoa repens, ladite substance active étant éventuellement incorporée au moins en partie dans lesdites phases lamellaires lipidiques hydratées ou les liposomes.

**13.** Utilisation d'une composition à base de phases lamellaires lipidiques hydratées ou de liposomes selon l'une des revendications 1 à 5, pour la préparation d'une composition cosmétique ou pharmaceutique, notamment dermatologique, destinée au traitement des cheveux, notamment pour favoriser la repousse des cheveux ou pour ralentir leur chute et pour prévenir l'apparition ou pour traiter les cheveux gris.

## Patentansprüche

## Patentansprüche für folgende Vertragsstaaten : AT, BE, CH, LI, DE, DK, GB, IT, LU, NL, SE

**1.** Zusammensetzung auf Basis von wasserhaltigen lamellaren Lipidphasen oder von Liposomen, dadurch gekennzeichnet, daß die wasserhaltigen lamellaren Lipidphasen oder die Liposome zumindest teilweise ein Labdanderivat der nachstehenden Formel I und/oder einen dieses enthaltenden Pflanzenextrakt enthalten:

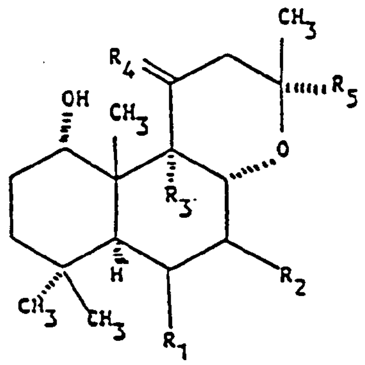

(I)

worin:

- $R_1$ und $R_2$ gleich oder verschieden sind und jeweils eine Hydroxygruppe oder eine Gruppe -O-CO-$R_6$ darstellen, worin $R_6$ eine Alkylgruppe mit 1 bis 7 Kohlenstoffatomen, eine Alkoxygruppe mit 1 bis 7 Kohlenstoffatomen oder eine Alkenylgruppe mit 1 bis 7 Kohlenstoffatomen, gegebenenfalls substituiert durch eine oder mehrere Hydroxygruppen oder

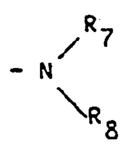

,

worin $R_7$ und $R_6$ jeweils ein Wasserstoffatom, eine Alkylgruppe mit 1 bis 4 Kohlenstoffatomen, wie Ethyl, sind oder aber zusammen und mit dem Stickstoffatom eine heterozyklische Gruppe, wie Piperidino, Morpholino, N'-Methyl-piperazino, bilden, darstellt;
- $R_3$ ein Wasserstoffatom oder eine Hydroxygruppe darstellt;
- $R_4$ ein Sauerstoffatom oder die Gruppe

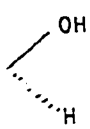

darstellt;
- $R_6$ eine Ethyl- oder Vinylgruppe darstellt.

2. Zusammensetzung nach Anspruch 1, dadurch gekennzeichnet, daß das Labdanderivat eine Verbindung der Formel (I) ist, worin:
- $R_1$ eine Hydroxygruppe ist;
- $R_2$ eine Hydroxygruppe oder eine Gruppe -O-CO-$R_6$ darstellt, worin $R_6$ eine Alkylgruppe mit 1 bis 4 Kohlenstoffatomen, eine Alkoxygruppe mit 1 bis 4 Kohlenstoffatomen oder eine Alkenylgruppe mit 1 bis 4 Kohlenstoffatomen, gegebenenfalls substituiert durch eine oder zwei Hydroxygruppen oder aber an einem Ende der Kette durch eine Gruppe

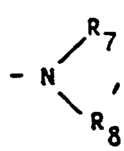

,

worin $R_7$ und $R_8$ jeweils eine Methyl- oder Ethylgruppe sind oder aber zusammen und mit dem Stickstoffatom eine heterozyklische Gruppe, wie Piperidino, Morpholino, N'-Methyl-piperazino, bilden, darstellt;
- $R_3$ eine Hydroxygruppe darstellt;
- $R_4$ ein Sauerstoffatom darstellt;

- $R_5$ eine Vinylgruppe darstellt.

3. Zusammensetzung nach Anspruch 2, dadurch gekennzeichnet, daß das Labdanderivat eine der folgenden Verbindungen ist:
   - Forskolin
   - 7-O-Deacetyl-forskolin
   - Coleforsin
   - 7-O-Deacetyl-7-β-O-propanoyl-forskolin
   - 7-O-Deacetyl-7-β-O-(ethoxycarbonyl)-forskolin
   - 7-O-Deacetyl-7-β-O-(propoxycarbonyl)-forskolin
   - 7-O-Deacetyl-7-β-O-(2,3-dihydroxy-propanoyl)-forskolin
   - 7-O-Deacetyl-7-β-O-(3-methyl-2-butenoyl)-forskolin
   - 7,0-Deacetyl-7-β-O-[4-(N'-methyl-piperazino)-butanoyl]-forskolin
   - 7-O-Deacetyl-7-β-O-(4-morpholino-butanoyl)-forskolin
   - 6-β-O-[Piperidino-propanoyl]-forskolin
   - 6-β-O-(Piperidino-acetyl)-forskolin.

4. Zusammensetzung nach Anspruch 1, dadurch gekennzeichnet, daß der ein Labdanderivat enthaltende Extrakt ein Extrakt von Coleus, insbesondere ein Extrakt von Coleus forskohlii, vorzugsweise ein Extrakt aus Wurzeln der Pflanze Coleus, ist.

5. Zusammensetzung nach Anspruch 4, dadurch gekennzeichnet, daß der Coleusextrakt ein organischer Coleusextrakt, insbesondere aus Wurzeln von Coleus, ist, welcher vorzugsweise durch ein Verfahren erhalten ist, das mindestens eine Extraktionsstufe mit Ethylacetat umfaßt.

6. Kosmetische oder pharmazeutische, insbesondere dermatologische Zusammensetzung, die insbesondere zur Begünstigung des Nachwachsens von Haaren bzw. zur Verzögerung des Haarausfalls oder zur Begünstigung der Pigmentierung der Epidermis oder zur Verhinderung des Auftretens von grauen Haaren oder zu deren Behandlung geeignet ist, dadurch gekennzeichnet, daß sie als aktives Ingrediens ein Labdanderivat der nachstehenden Formel I und/oder einen dieses enthaltenden Pflanzenextrakt, zumindest teilweise in wasserhaltigen lamellaren Lipidphasen oder in Liposomen eingearbeitet, aufweist:

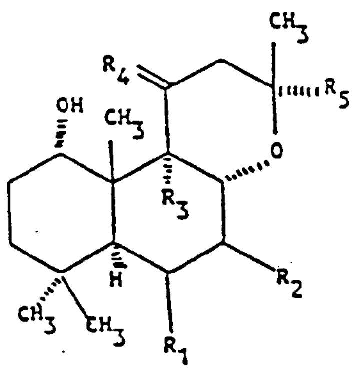

(I)

worin

- die Gruppen $R_1$ und $R_2$ gleich oder verschieden sind und eweils eine Hydroxygruppe oder eine Gruppe -O-CO-$R_6$ darstellen, worin $R_6$ eine Alkylgruppe mit 1 bis 7 Kohlenstoffatomen, eine Alkoxygruppe mit 1 bis 7 Kohlenstoffatomen oder eine Alkenylgruppe mit 1 bis 7 Kohlenstoffatomen, gegebenenfalls substituiert durch eine oder mehrere Hydroxygruppen oder

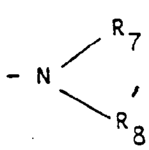

worin $R_7$ und $R_5$ jeweils ein Wasserstoffatom, eine Alkylgruppe mit 1 bis 4 Kohlenstoffatomen, wie

Methyl oder Ethyl, sind oder aber zusammen und mit dem Stickstoffatom eine heterozyklische Gruppe, wie Piperidino, Morpholino, N'-Methyl-piperazino, bilden, darstellt;
- $R_3$ ein Wasserstoffatom oder eine Hydroxygruppe darstellt;
- $R_4$ ein Sauerstoffatom oder die Gruppe

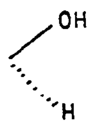

darstellt;
- $R_5$ eine Ethyl- oder Vinylgruppe darstellt.

7. Kosmetische oder pharmazeutische, insbesondere dermatologische Zusammensetzung nach Anspruch 6, dadurch gekennzeichnet, daß das Labdanderivat eine Verbindung der Formel (I) ist, worin:
   - $R_1$ eine Hydroxygruppe ist;
   - $R_2$ eine Hydroxygruppe oder eine Gruppe -O-CO-$R_6$ darstellt, worin $R_5$ eine Alkylgruppe mit 1 bis 4 Kohlenstoffatomen, eine Alkoxygruppe mit 1 bis 4 Kohlenstoffatomen oder eine Alkenylgruppe mit 1 bis 4 Kohlenstoffatomen, gegebenenfalls substituiert durch eine oder zwei Hydroxygruppen oder aber an einem Ende der Kette durch eine Gruppe

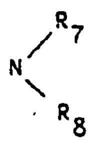

,

     worin $R_7$ und $R_8$ jeweils eine Methyl- oder Ethylgruppe sind oder aber zusammen und mit dem Stickstoffatom eine heterozyklische Gruppe, wie Piperidino, Morpholino, N'-Methyl-piperazino, bilden, darstellt;
   - $R_3$ eine Hydroxygruppe darstellt;
   - $R_4$ ein Sauerstoffatom darstellt;
   - $R_5$ eine Vinylgruppe darstellt.

8. Kosmetische oder pharmazeutische, insbesondere dermatologische Zusammensetzung nach einem der Ansprüche 6 und 7, dadurch gekennzeichnet, daß das Labdanderivat eine der folgenden Verbindungen ist:
   - Forskolin
   - 7-O-Deacetyl-forskolin
   - Coleforsin
   - 7-O-Deacetyl-7-β-O-propanoyl-forskolin
   - 7-O-Deacetyl-7-β-O-(ethoxycarbonyl)-forskolin
   - 7-O-Deacetyl-7-β-O-(propoxycarbonyl)-forskolin
   - 7-O-Deacetyl-7-β-O-(2,3-dihydroxy-propanoyl)-forskolin
   - 7-O-Deacetyl-7-β-O-(3-methyl-2-butenoyl)-forskolin
   - 7,0-Deacetyl-7-β-O-[4-(N'-methyl-piperazino)-butanoyl]-forskolin
   - 7-O-Deacetyl-7-β-O-(4-morpholino-butanoyl)-forskolin
   - 6-β-O-[3-Piperidino-propanoyl]-forskolin
   - 6-β-O-(Piperidino-acetyl)-forskolin.

9. Kosmetische oder pharmazeutische, insbesondere dermatologische Zusammensetzung nach Anspruch 6, dadurch gekennzeichnet, daß der ein Labdanderivat enthaltende Extrakt ein Extrakt von Coleus, insbesondere ein Extrakt von Coleus forskohlii, vorzugsweise ein Extrakt aus Wurzeln der Pflanze Coleus, ist.

10. Kosmetische oder pharmazeutische, insbesondere dermatologische Zusammensetzung nach Anspruch 9, dadurch gekennzeichnet, daß der Coleusextrakt ein organischer Coleusextrakt, insbesondere aus Wurzeln von Coleus, ist, welcher vorzugsweise durch ein Verfahren erhalten ist, das mindestens eine Extraktionsstufe mit Ethylacetat umfaßt.

11. Kosmetische oder pharmazeutische, insbesondere dermatologische Zusammensetzung nach einem der Ansprüche 6 bis 10, dadurch gekennzeichnet, daß die Konzentration an Labdanderivat oder an dieses enthaltendem Extrakt, zumindest teilweise in wasserhaltigen lamellaren Lipidphasen oder in Liposomen eingearbeitet, zwischen 0,0001 und 1 Gew.%, vorzugsweise zwischen 0,01 und 0,1 Gew.%, bezogen auf das Gesamtgewicht der Zusammensetzung, beträgt.

12. Kosmetische oder pharmazeutische, insbesondere dermatologische Zusammensetzung nach einem der Ansprüche 6 bis 11, dadurch gekennzeichnet, daß sie außerdem mindestens eine weitere aktive Substanz in einer wirksamen Konzentration aufweist, die ausgewählt ist aus Xanthinen, Vitaminen, insbesondere Vitamin B, Tyrosin oder Derivaten davon, wie beispielsweise Glukosetyrosinat, Chinin oder Derivaten davon, Rötungsmittel, wie Methylnikotinat, einem Überstand an Papillenfibroblastenkultur, Keratinhydrolysaten, Spurenelementen, wie Zink, Selen, Kupfer, 5-α-Reductase-Inhibitoren, wie Progesteron, Cyproteronacetat, Minoxidil, Azelainsäure und Derivaten davon, einem 4-Methyl-4-azasteroid, insbesondere 17-β-N,N-Diethylcarbamoyl-4-methyl-4-aza-5-α-androstan-3-on, oder auch einem Extrakt von Serenoa repens, welche aktive Substanz gegebenenfalls zumindest teilweise in die wasserhaltigen lamellaren Lipidphasen oder die Liposome eingearbeitet ist.

13. Verwendung einer Zusammensetzung auf Basis von wasserhaltigen lamellaren Lipidphasen oder von Liposomen nach einem der Ansprüche 1 bis 5 zur Herstellung einer kosmetischen oder pharmazeutischen, insbesondere dermatologischen Zusammensetzung, die zur Behandlung von Haaren, insbesondere zur Begünstigung des Nachwachsens der Haare bzw. zur Verzögerung des Haarausfalls und zur Verhinderung des Auftretens von grauen Haaren oder zu deren Behandlung bestimmt ist.

**Patentansprüche für folgenden Vertragsstaat : ES**

1. Verfahren zur Herstellung einer Zusammensetzung auf Basis von wasserhaltigen lamellaren Lipidphasen oder von Liposomen, dadurch gekennzeichnet, daß man in die wasserhaltigen lamellaren Lipidphasen oder die Liposome zumindest teilweise ein Labdanderivat der nachstehenden Formel I und/oder einen dieses enthaltenden Pflanzenextrakt einarbeitet:

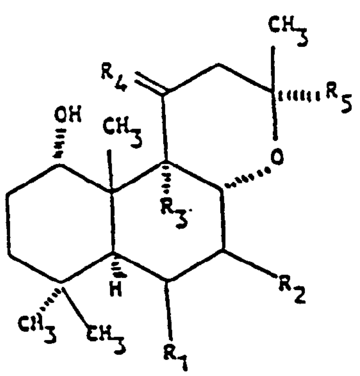

(I)

worin:

- $R_1$ und $R_2$ gleich oder verschieden sind und jeweils eine Hydroxygruppe oder eine Gruppe -O-CO-$R_6$ darstellen, worin $R_6$ eine Alkylgruppe mit 1 bis 7 Kohlenstoffatomen, eine Alkoxygruppe mit 1 bis 7 Kohlenstoffatomen oder eine Alkenylgruppe mit 1 bis 7 Kohlenstoffatomen, gegebenenfalls substituiert durch eine oder mehrere Hydroxygruppen oder

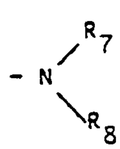

,

worin $R_7$ und $R_8$ jeweils ein Wasserstoffatom, eine Alkylgruppe mit 1 bis 4 Kohlenstoffatomen, wie Ethyl, sind oder aber zusammen und mit dem Stickstoffatom eine heterozyklische Gruppe, wie Piperidino, Morpholino, N'-Methyl-piperazino, bilden, darstellt;

- $R_3$ ein Wasserstoffatom oder eine Hydroxygruppe darstellt;
- $R_4$ ein Sauerstoffatom oder die Gruppe

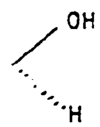

darstellt;

- $R_8$ eine Ethyl- oder Vinylgruppe darstellt.

**2.** Verfahren nach Anspruch 1, dadurch gekennzeichnet, daß man als Labdanderivat eine Verbindung der Formel (I) einarbeitet, worin:

- $R_1$ eine Hydroxygruppe ist;
- $R_2$ eine Hydroxygruppe oder eine Gruppe -O-CO-$R_6$ darstellt, worin $R_8$ eine Alkylgruppe mit 1 bis 4 Kohlenstoffatomen, eine Alkoxygruppe mit 1 bis 4 Kohlenstoffatomen oder eine Alkenylgruppe mit 1 bis 4 Kohlenstoffatomen, gegebenenfalls substituiert durch eine oder zwei Hydroxygruppen oder aber an einem Ende der Kette durch eine Gruppe

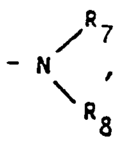

worin $R_7$ und $R_8$ jeweils eine Methyl- oder Ethylgruppe sind oder aber zusammen und mit dem Stickstoffatom eine heterozyklische Gruppe, wie Piperidino, Morpholino, N'-Methyl-piperazino, bilden, darstellt;

- $R_3$ eine Hydroxygruppe darstellt;
- $R_4$ ein Sauerstoffatom darstellt;
- $R_8$ eine Vinylgruppe darstellt.

**3.** Verfahren nach Anspruch 2, dadurch gekennzeichnet, daß man als Labdanderivat eine der folgenden Verbindungen einarbeitet:

- Forskolin
- 7-O-Deacetyl-forskolin
- Coleforsin
- 7-O-Deacetyl-7-β-O-propanoyl-forskolin
- 7-O-Deacetyl-7-β-O-(ethoxycarbonyl)-forskolin
- 7-O-Deacetyl-7-β-O-(propoxycarbonyl)-forskolin
- 7-O-Deacetyl-7-β-O-(2,3-dihydroxy-propanoyl)-forskolin
- 7-O-Deacetyl-7-β-O-[4-methyl-2-butenoyl)-forskolin
- 7,0-Deacetyl-7-β-O-(4-(N'-methyl-piperazino)-butanoyl]forskolin
- 7-O-Deacetyl-7-β-0-(4-morpholino-butanoyl)-forskolin
- 6-β-O-[3- Piperidino-propanoyl]-forskolin
- 6-β-O-(Piperidino-acetyl)-forskolin.

**4.** Verfahren nach Anspruch 1, dadurch gekennzeichnet daß man als ein Labdanderivat enthaltenden Extrakt einen Extrakt von Coleus, insbesondere einen Extrakt von Coleus forskohlii, vorzugsweise einen Extrakt aus Wurzeln der Pflanze Coleus, einarbeitet.

**5.** Verfahren nach Anspruch 4, dadurch gekennzeichnet, daß man als Coleusextrakt einen organischen Coleusextrakt, insbesondere aus Wurzeln von Coleus, einarbeitet, welcher vorzugsweise durch ein Verfahren erhalten wird, das mindestens eine Extraktionsstufe mit Ethylacetat umfaßt.

6. Verfahren zur Herstellung einer kosmetischen oder pharmazeutischen, insbesondere dermatologischen Zusammensetzung, die insbesondere zur Begünstigung des Nachwachsens von Haaren bzw. zur Verzögerung des Haarausfalls oder zur Begünstigung der Pigmentierung der Epidermis oder zur Verhinderung des Auftretens von grauen Haaren oder zu deren Behandlung geeignet ist, dadurch gekennzeichnet, daß man als aktives Ingrediens ein Labdanderivat der nachstehenden Formel I und/oder einen dieses enthaltenden Pflanzenextrakt, zumindest teilweise in wasserhaltigen lamellaren Lipidphasen oder in Liposomen eingearbeitet, verwendet:

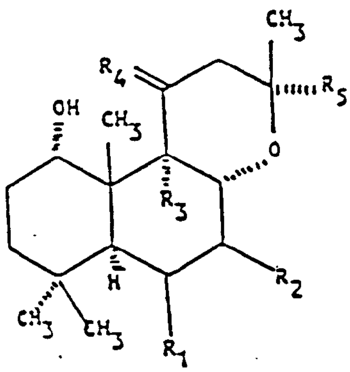

(I)

worin

- die Gruppen $R_1$ und $R_2$ gleich oder verschieden sind und jeweils eine Hydroxygruppe oder eine Gruppe -O-CO-$R_6$ darstellen, worin $R_6$ eine Alkylgruppe mit 1 bis 7 Kohlenstoffatomen, eine Alkoxygruppe mit 1 bis 7 Kohlenstoffatomen oder eine Alkenylgruppe mit 1 bis 7 Kohlenstoffatomen, gegebenenfalls substituiert durch eine oder mehrere Hydroxygruppen oder

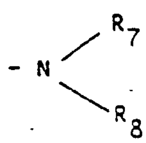,

worin $R_7$ und $R_8$ jeweils ein Wasserstoffatom, eine Alkylgruppe mit 1 bis 4 Kohlenstoffatomen, wie Methyl oder Ethyl, sind oder aber zusammen und mit dem Stickstoffatom eine heterozyklische Gruppe, wie Piperidino, Morpholino, N'-Methyl-piperazino, bilden, darstellt;
- $R_3$ ein Wasserstoffatom oder eine Hydroxygruppe darstellt;
- $R_4$ ein Sauerstoffatom oder die Gruppe

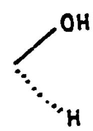

darstellt;
- $R_8$ eine Ethyl- oder Vinylgruppe darstellt.

7. Verfahren nach Anspruch 6, dadurch gekennzeichnet, daß man als Labdanderivat eine Verbindung der Formel (I) verwendet, worin:
- $R_1$ eine Hydroxygruppe ist;
- $R_2$ eine Hydroxygruppe oder eine Gruppe -O-CO-$R_6$ darstellt, worin $R_8$ eine Alkylgruppe mit 1 bis 4 Kohlenstoffatomen, eine Alkoxygruppe mit 1 bis 4 Kohlenstoffatomen oder eine Alkenylgruppe mit 1 bis 4 Kohlenstoffatomen, gegebenenfalls substituiert durch eine oder zwei Hydroxygruppen oder aber an einem Ende der Kette durch eine Gruppe

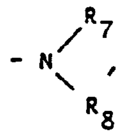
,

worin $R_7$ und $R_8$ jeweils eine Methyl- oder Ethylgruppe sind oder aber zusammen und mit dem Stickstoffatom eine heterozyklische Gruppe, wie Piperidino, Morpholino, N'-Methyl-piperazino, bilden, darstellt;
- $R_3$ eine Hydroxygruppe darstellt;
- $R_4$ ein Sauerstoffatom darstellt;
- $R_5$ eine Vinylgruppe darstellt.

**8.** Verfahren nach einem der Ansprüche 6 und 7, dadurch gekennzeichnet, daß man als Labdanderivat eine der folgenden Verbindungen verwendet:
- Forskolin
- 7-O-Déacetyl-forskolin
- Coleforsin
- 7-O-Deacetyl-7-β-O-propanoyl-forskolin
- 7-O-Deacetyl-7-β-O-(ethoxycarbonyl)-forskolin
- 7-O-Deacetyl-7-β-O-(propoxycarbonyl)-forskolin
- 7-O-Deacetyl-7-β-O-(2,3-dihydroxy-propanoyl)-forskolin
- 7-O-Deacetyl-7-β-O-(3-methyl-2-butenoyl)-forskolin
- 7,0-Deacetyl-7-β-O-(4-(N'-methyl-piperazino)-butanoyl]forskolin
- 7-O-Deacetyl-7-β-O-(4-morpholino-butanoyl)-forskolin
- 6-β-O-(3-Piperidino-propanoyl]-forskolin
- 6-β-O-(Piperidino-acetyl)-forskolin

**9.** Verfahren nach Anspruch 6, dadurch gekennzeichnet, daß man als ein Labdanderivat enthaltenden Extrakt einen Extrakt von Coleus, insbesondere einen Extrakt von Coleus forskohlii, vorzugsweise einen Extrakt aus Wurzeln der Pflanze Coleus, verwendet.

**10.** Verfahren nach Anspruch 9, dadurch gekennzeichnet, daß man als Coleusextrakt einen organischen Coleusextrakt, insbesondere aus Wurzeln von Coleus, verwendet, welcher vorzugsweise durch ein Verfahren erhalten wird, das mindestens eine Extraktionsstufe mit Ethylacetat umfaßt.

**11.** Verfahren nach einem der Ansprüche 6 bis 10, dadurch gekennzeichnet, daß man eine Konzentration an Labdanderivat oder an dieses enthaltendem Extrakt, zumindest teilweise in wasserhaltigen lamellaren Lipidphasen oder in Liposomen eingearbeitet, zwischen 0,0001 und 1 Gew.%, vorzugsweise zwischen 0,01 und 0,1 Gew.%, bezogen auf das Gesamtgewicht der Zusammensetzung, verwendet.

**12.** Verfahren nach einem der Ansprüche 6 bis 11, dadurch gekennzeichnet, daß man außerdem mindestens eine weitere aktive Substanz in einer wirksamen Konzentration zugibt, die ausgewählt ist aus Xanthinen, Vitaminen, insbesondere Vitamin B, Tyrosin oder Derivaten davon, wie beispielsweise Glukosetyrosinat, Chinin oder Derivaten davon, Rötungsmittel, wie Methylnikotinat, einem Überstand an Papillenfibroblastenkultur, Keratinhydrolysaten, Spurenelementen, wie Zink, Selen, Kupfer, 5-α-Reductase-Inhibitoren, wie Progesteron, Cyproteronacetat, Minoxidil, Azelainsäure und Derivaten davon, einem 4-Methyl-4-azasteroid, insbesondere 17-β-N,N-Diethylcarbamoyl-4-methyl-4-aza-5-α-androstan-3-on, oder auch einem Extrakt von Serenoa repens, welche aktive Substanz gegebenenfalls zumindest teilweise in die wasserhaltigen lamellaren Lipidphasen oder die Liposome eingearbeitet wird.

**13.** Verwendung einer Zusammensetzung auf Basis von wasserhaltigen lamellaren Lipidphasen oder von Liposomen nach einem der Ansprüche 1 bis 5 zur Herstellung einer kosmetischen oder pharmazeutischen, insbesondere dermatologischen Zusammensetzung, die zur Behandlung der Haare, insbesondere zur Begünstigung des Nachwachsens von Haaren bzw. zur Verzögerung des Haarausfalls und zur Verhinderung des Auftretens von grauen Haaren oder zu deren Behandlung bestimmt ist.

## Claims

### Claims for the following Contracting States : AT, BE, CH, LI, DE, DK, GB, IT, LU, NL, SE

1. Composition based on hydrated lipidic lamellar phases or liposomes, characterized in that said hydrated lipidic lamellar phases or said liposomes contain at least in part a derivative of labdane, of the formula I hereunder, and/or a plant extract containing it:

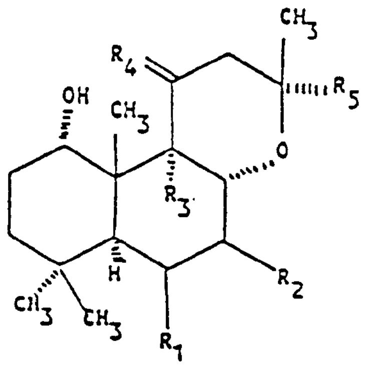

(I)

in which:

- $R_1$ and $R_2$ are identical or different and each represent a hydroxy group or a -O-CO-$R_6$ group in which $R_6$ represents an alkyl radical having from 1 to 7 atoms of carbon, alkoxy radical having from 1 to 7 atoms of carbon or alkenyl radical having from 1 to 7 atoms of carbon, possibly substituted by one or more hydroxy or

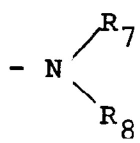

groups
in which $R_7$ and $R_6$ each represent an atom of hydrogen, an alkyl radical having from 1 to 4 atoms of carbon such as ethyl or represent together and with the atom of nitrogen a heterocyclic radical such as piperidino, morpholino, N'-methyl-piperazino;
- $R_3$ represents an atom of hydrogen or a hydroxy group;
- $R_4$ represents an atom of oxygen or the assembly

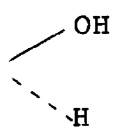

- $R_6$ represents an ethyl or vinyl radical.

2. Composition according to claim 1, characterized in that the labdane derivative mentioned above is a compound of formula I in which:
   - $R_1$ represents the hydroxy group;
   - $R_2$ represents a hydroxy group or a -O-CO-$R_6$ group in which $R_6$ represents an alkyl radical having from 1 to 4 atoms of carbon, an alkoxy radical having from 1 to 4 atoms of carbon or an alkenyl radical having from 1 to 4 atoms of carbon, possibly substituted by one or two hydroxy groups or, at one chain end, by a

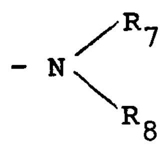

group
in which $R_7$ and $R_8$ each represent a methyl or ethyl radical or represent together and with the atom of nitrogen a heterocyclic radical such as piperidino, morpholino, N'-methylpiperazino;
- $R_3$ represents the hydroxy group,
- $R_4$ represents an atom of oxygen,
- $R_5$ represents the vinyl radical.

3. Composition according to claim 2, characterized in that the labdane derivative mentioned above is one of the following compounds:
   - forskoline
   - 7-0-deacetyl-forskoline
   - coleforsine
   - 7-0-deacetyl-7-β-0-propanoyl-forskoline
   - 7-0-deacetyl-7-β-0-(ethoxycarbonyl)-forskoline
   - 7-0-deacetyl-7-β-O-(propoxycarbonyl)-forskoline
   - 7-0-deacetyl-7-β-O-(2,3-dihydroxy-propanoyl)-forskoline
   - 7-0-deacetyl-7-β-O-(3-methyl-2-butenoyl)-forskoline
   - 7,0-deacetyl-7-β-0-[4-(N'-methyl-piperazino)-butanoyl]
   - forskoline
   - 7-0-deacetyl-7-β-O-(4-morpholino-butanoyl)-forskoline
   - 6-β-0-[3-piperidino-propanoyl]-forskoline
   - 6-β-0-(piperidino-acetyl)-forskoline

4. Composition according to claim 1, characterized in that the extract containing a labdane derivative mentioned above is an extract of Coleus, in particular an extract of Coleus forskohlii, preferably an extract of roots of the plant Coleus.

5. Composition according to claim 4, characterized in that the Coleus extract mentioned above is an organic extract of Coleus, particularly of roots of Coleus, preferably obtained by a process including at least one step of extraction with ethyl acetate.

6. Cosmetic or pharmaceutical, particularly dermatological composition, intended in particular to promote regrowth of the hair or to reduce hair drop, or to promote pigmentation of the epidermis or to prevent gray hair from appearing or to treat gray hair, characterized in that it comprises, by way of active ingredient, a derivative of labdane, of the formula I hereunder, and/or an extract of plant containing it, at least in part incorporated in hydrated lipidic lamellar phases or in liposomes:

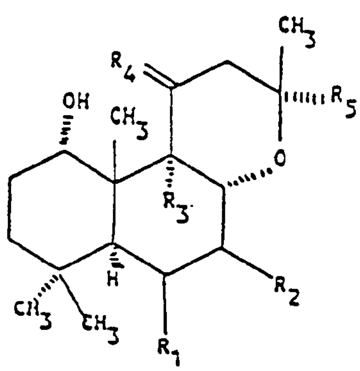

(I)

in which:
- the groups $R_1$ a,d $R_2$ are identical or different and each represent a hydroxy group or a -O-CO-$R_5$

group in which $R_6$ represents an alkyl radical having from 1 to 7 atoms of carbon, alkoxy radical having from 1 to 7 atoms of carbon or alkenyl radical having from 1 to 7 atoms of carbon, possibly substituted by one or more hydroxy or

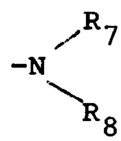

groups
in which $R_7$ and $R_8$ each represent an atom of hydrogen, an alkyl radical having 1 to 4 atoms of carbon such as methyl or ethyl or represent together and with the atom of nitrogen a heterocyclic radical such as piperidino, morpholino, N'-methyl-piperazino;
- $R_3$ represents an atom of hydrogen or a hydroxy group;
- $R_4$ represents an atom of oxygen or the assembly

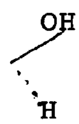

- $R_5$ represents an ethyl or vinyl radical.

**7.** Cosmetic or pharmaceutical, particularly dermatological composition, according to claim 6, characterized in that the labdane derivative mentioned above is a compound of formula I mentioned above in which:
- $R_1$ represents the hydroxy group;
- $R_2$ represents a hydroxy group or a -O-CO-$R_6$ group in which $R_5$ represents an alkyl radical having from 1 to 4 atoms of carbon, an alkoxy radical having from 1 to 4 atoms of carbon or an alkenyl radical having from 1 to 4 atoms of carbon, possibly substituted by one or two hydroxy groups or, at one chain end, by a

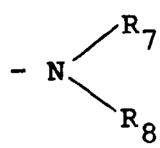

group in which
$R_7$ and $R_8$ each represent a methyl or ethyl radical or represent together and with the neighbouring atom of nitrogen a heterocyclic radical such as piperidino, morpholino, N'-methylpiperazino;
- $R_3$ represents the hydroxy group,
- $R_4$ represents an atom of oxygen,
- $R_5$ represents the vinyl radical.

**8.** Cosmetic or pharmaceutical, particularly dermatological composition according to one of claims 6 and 7, characterized in that the labdane derivative is one of the following compounds:
- forskoline
- 7-0-deacetyl-forskoline
- coleforsine
- 7-0-deacetyl-7-β-0-propanoyl-forskoline
- 7-0-deacetyl-7-β-0-(ethoxycarbonyl)-forskoline
- 7-0-deacetyl-7-β-0-(propoxycarbonyl)-forskoline
- 7-0-deacetyl-7-β-0-(2,3-dihydroxy-propanoyl)-forskoline
- 7-0-deacetyl-7-β-0-(3-methyl-2-butenoyl)-forskoline
- 7,0-deacetyl-7-β-0-[4-(N'-methyl-piperazino)-butanoyl]-forskoline

- 7-0-deacetyl-7-β-O-(4-morpholino-butanoyl)-forskoline
- 6-β-O-[3-piperidino-propanoyl]-forskoline
- 6-β-0-(piperidino-acetyl)-forskoline.

**9.** Cosmetic or pharmaceutical, particularly dermatological composition according to claim 6, characterized in that the extract containing a derivative of labdane mentioned above is an extract of Coleus, in particular an extract of Coleus forskohlii, preferably an extract of roots of Coleus.

**10.** Cosmetic or pharmaceutical, particularly dermatological composition, according to claim 9, characterized in that the extract of Coleus mentioned above is an organic extract of Coleus, particularly of roots of Coleus, preferably obtained by a process comprising at least one step of extraction with ethyl acetate.

**11.** Cosmetic or pharmaceutical, particularly dermatological composition, according to one of claims 6 to 10, characterized in that the concentration of derivative of labdane mentioned above or of extract containing it, incorporated at least in part in hydrated lipidic lamellar phases or in liposomes, is included between 0.0001% and 1% by weight, preferably between 0.01% and 0.1% by weight, with respect to the total weight of the composition.

**12.** Cosmetic or pharmaceutical, particularly dermatological composition according to one of claims 6 to 11, characterized in that it comprises in addition at least one other active substance at an efficient concentration, selected from xanthines, vitamins, particularly vitamin B's, tyrosine or its derivatives such as for example glucose tyrosinate, quinine or its derivatives, rubefacients such as methyl nicotinate, a supernatant of culture of fibroblasts of papillae, keratin hydrolysates, oligo-elements such as zinc, selenium, copper, 5-α-reductase inhibitors such as: progesterone, cyproterone acetate, Minoxidil, azelaic acid and its derivatives, a 4-methyl-4-azasteroid, in particular 17-β-N,N-diethylcarbamoyl-4-methyl-4-aza-5-α-androstan-3-one or an extract of Serenoa repens, said active substance possibly being incorporated at least in part in said hydrated lipidic lamellar phases or the liposomes.

**13.** Use of a composition based on hydrated lipidic lamellar phases or of liposomes according to one of claims 1 to 5, for the preparation of a cosmetic or pharmaceutical, particularly dermatological composition intended for the treatment of the hair, particularly for promoting regrowth of the hair or for reducing hair drop and for preventing gray hair from appearing or for treating gray hair.

**Claims for the following Contracting State : ES**

**1.** A process for the preparation of a composition based on hydrated lipidic lamellar phases or liposomes, characterized in that a derivative of labdane of the formula I hereunder, and/or a plant extract containing it is incorporated at least in part in said hydrated lipidic lamellar phases or said liposomes:

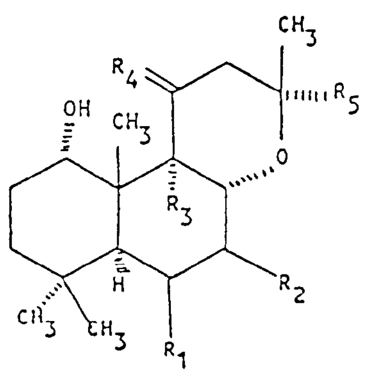

(I)

in which:

- $R_1$ and $R_2$ are identical or different and each represent a hydroxy group or a -O-CO-$R_6$ group in which $R_6$ represents an alkyl radical having from 1 to 7 atoms of carbon, alkoxy radical having from 1 to 7 atoms of carbon or alkenyl radical having from 1 to 7 atoms of carbon, possibly substituted by one or more hydroxy or

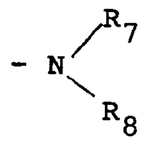

groups
in which $R_7$ and $R_8$ each represent an atom of hydrogen, an alkyl radical having from 1 to 4 atoms of carbon such as ethyl or represent together and with the atom of nitrogen a heterocyclic radical such as piperidino, morpholino, N'-methyl-piperazino;

- $R_3$ represents an atom of hydrogen or a hydroxy group;
- $R_4$ represents an atom of oxygen or the assembly

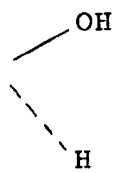

- $R_8$ represents an ethyl or vinyl radical.

**2.** A process according to claim 1, characterized in that as the labdane derivative mentioned above a compound of formula I is incorporated, in which:

- $R_1$ represents the hydroxy group;
- $R_2$ represents a hydroxy group or a -O-CO-$R_6$ group in which $R_8$ represents an alkyl radical having from 1 to 4 atoms of carbon, an alkoxy radical having from 1 to 4 atoms of carbon or an alkenyl radical having from 1 to 4 atoms of carbon, possibly substituted by one or two hydroxy groups or, at one chain end, by a

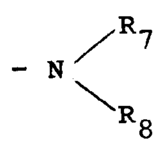

group
in which $R_7$ and $R_8$ each represent a methyl or ethyl radical or represent together and with the atom of nitrogen a heterocyclic radical such as piperidino, morpholino, N'-methylpiperazino;

- $R_3$ represents the hydroxy group,
- $R_4$ represents an atom of oxygen,
- $R_8$ represents the vinyl radical.

**3.** A process according to claim 2, characterized in that as the labdane derivative mentioned above one of the following compounds is incorporated :

- forskoline
- 7-0-deacetyl-forskoline
- coleforsine
- 7-0-deacetyl-7-β-0-propanoyl-forskoline
- 7-0-deacetyl-7-β-O-(ethoxycarbonyl)-forskoline
- 7-0-deacetyl-7-β-O-(propoxycarbonyl)-forskoline
- 7-0-deacetyl-7-β-O-(2,3-dihydroxy-propanoyl)-forskoline
- 7-0-deacetyl-7-β-O-(3-methyl-2-butenoyl)-forskoline
- 7,0-deacetyl-7-β-0-[4-(N'-methyl-piperazino)-butanoyl]
- forskoline
- 7-0-deacetyl-7-β-O-(4-morpholino-butanoyl)-forskoline
- 6-β-0-[3-piperidino-propanoyl]-forskoline
- 6-β-0-(piperidino-acetyl)-forskoline

4. A process according to claim 1, characterized in that as the extract containing a labdane derivative mentioned above, an extract of Coleus is incorporated, in particular an extract of Coleus forskohlii, preferably an extract of roots of the plant Coleus.

5. A process according to claim 4, characterized in that as the Coleus extract mentioned above, an organic extract of Coleus is incorporated, particularly of roots of Coleus, preferably obtained by a process including at least one step of extraction with ethyl acetate.

6. A process for the preparation of a cosmetic or pharmaceutical, particularly dermatological composition, intended in particular to promote regrowth of the hair or to reduce hair drop, or to promote pigmentation of the epidermis or to prevent gray hair from appearing or to treat gray hair, characterized in that, by way of active ingredient, a derivative of labdane, of the formula I hereunder, and/or an extract of plant containing it is used, which is incorporated at least in part in hydrated lipidic lamellar phases or in liposomes:

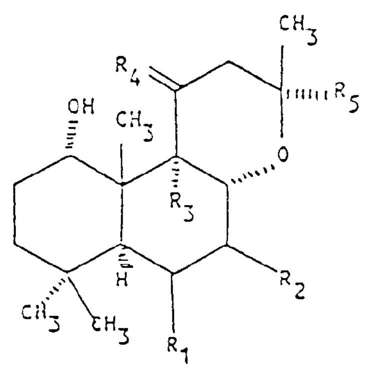

(I)

in which:

- the groups $R_1$ and $R_2$ are identical or different and each represent a hydroxy group or a -O-CO-$R_6$ group in which $R_6$ represents an alkyl radical having from 1 to 7 atoms of carbon, alkoxy radical having from 1 to 7 atoms of carbon or alkenyl radical having from 1 to 7 atoms of carbon, possibly substituted by one or more hydroxy or

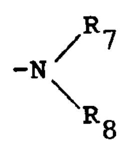

groups
in which $R_7$ and $R_8$ each represent an atom of hydrogen, an alkyl radical having 1 to 4 atoms of carbon such as methyl or ethyl or represent together and with the atom of nitrogen a heterocyclic radical such as piperidino, morpholino, N'-methyl-piperazino;
- $R_3$ represents an atom of hydrogen or a hydroxy group;
- $R_4$ represents an atom of oxygen or the assembly

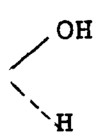

- $R_6$ represents an ethyl or vinyl radical.

7. A process according to claim 6, characterized in that a compound of formula I mentioned above is used as the labdane derivative mentioned above, in which :
- $R_1$ represents the hydroxy group;
- $R_2$ represents a hydroxy group or a -O-CO-$R_6$ group in which $R_6$ represents an alkyl radical having

from 1 to 4 atoms of carbon, an alkoxy radical having from 1 to 4 atoms of carbon or an alkenyl radical having from 1 to 4 atoms of carbon, possibly substituted by one or two hydroxy groups or, at one chain end, by a

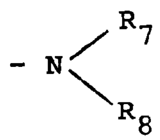

group in which
$R_7$ and $R_8$ each represent a methyl or ethyl radical or represent together and with the neighbouring atom of nitrogen a heterocyclic radical such as piperidino, morpholino, N'-methylpiperazino;
- $R_3$ represents the hydroxy group,
- $R_4$ represents an atom of oxygen,
- $R_5$ represents the vinyl radical.

**8.** A process according to one of claims 6 and 7, characterized in that one of the following compounds is used as the labdane derivative mentioned above:
- forskoline
- 7-0-deacetyl-forskoline
- coleforsine
- 7-0-deacetyl-7-β-0-propanoyl-forskoline
- 7-0-deacetyl-7-β-0-(ethoxycarbonyl)-forskoline
- 7-0-deacetyl-7-β-0-(propoxycarbonyl)-forskoline
- 7-0-deacetyl-7-β-0-(2,3-dihydroxy-propanoyl)-forskoline
- 7-0-deacetyl-7-β-0-(3-methyl-2-butenoyl)-forskoline
- 7,0-deacetyl-7-β-0-[4-(N'-methyl-piperazino)-butanoyl]-forskoline
- 7-0-deacetyl-7-β-O-(4-morpholino-butanoyl)-forskoline
- 6-β-O-[3-piperidino-propanoyl]-forskoline
- 6-β-0-(piperidino-acetyl)-forskoline.

**9.** A process according to claim 6, characterized in that an extract of Coleus, in particular an extract of Coleus forskohlii, preferably an extract of roots of Coleus is used as the extract containing a derivative of labdane mentioned above.

**10.** A process according to claim 9, characterized in that an organic extract of Coleus, particularly of roots of Coleus, preferably obtained by a process comprising at least one step of extraction with ethyl acetate is used as the extract of Coleus mentioned above.

**11.** A process according to one of claims 6 to 10, characterized in that a concentration of derivative of labdane mentioned above or of extract containing it, incorporated at least in part in hydrated lipidic lamellar phases or in liposomes, included between 0.0001% and 1% by weight, preferably between 0.01% and 0.1% by weight, with respect to the total weight of the composition, is used.

**12.** A process according to one of claims 6 to 11, characterized in that in addition at least one other active substance at an efficient concentration is added, selected from xanthines, vitamins, particularly vitamin B's, tyrosine or its derivatives such as for example glucose tyrosinate, quinine or its derivatives, rubefacients such as methyl nicotinate, a supernatant of culture of fibroblasts of papillae, keratin hydrolysates, oligo-elements such as zinc, selenium, copper, 5-α-reductase inhibitors such as: progesterone, cyproterone acetate, Minoxidil, azelaic acid and its derivatives, a 4-methyl-4-azasteroid, in particular 17-β-N,N-diethylcarbamoyl-4-methyl-4-aza-5-α-androstan-3-one or an extract of Serenoa repens, said active substance possibly being incorporated at least in part in said hydrated lipidic lamellar phases or the liposomes.

**13.** Use of a composition based on hydrated lipidic lamellar phases or of liposomes according to one of claims 1 to 5, for the preparation of a cosmetic or pharmaceutical, particularly dermatological composition intended for the treatment of the hair, particularly for promoting regrowth of the hair or for reducing hair drop and for preventing gray hair from appearing or for treating gray hair.

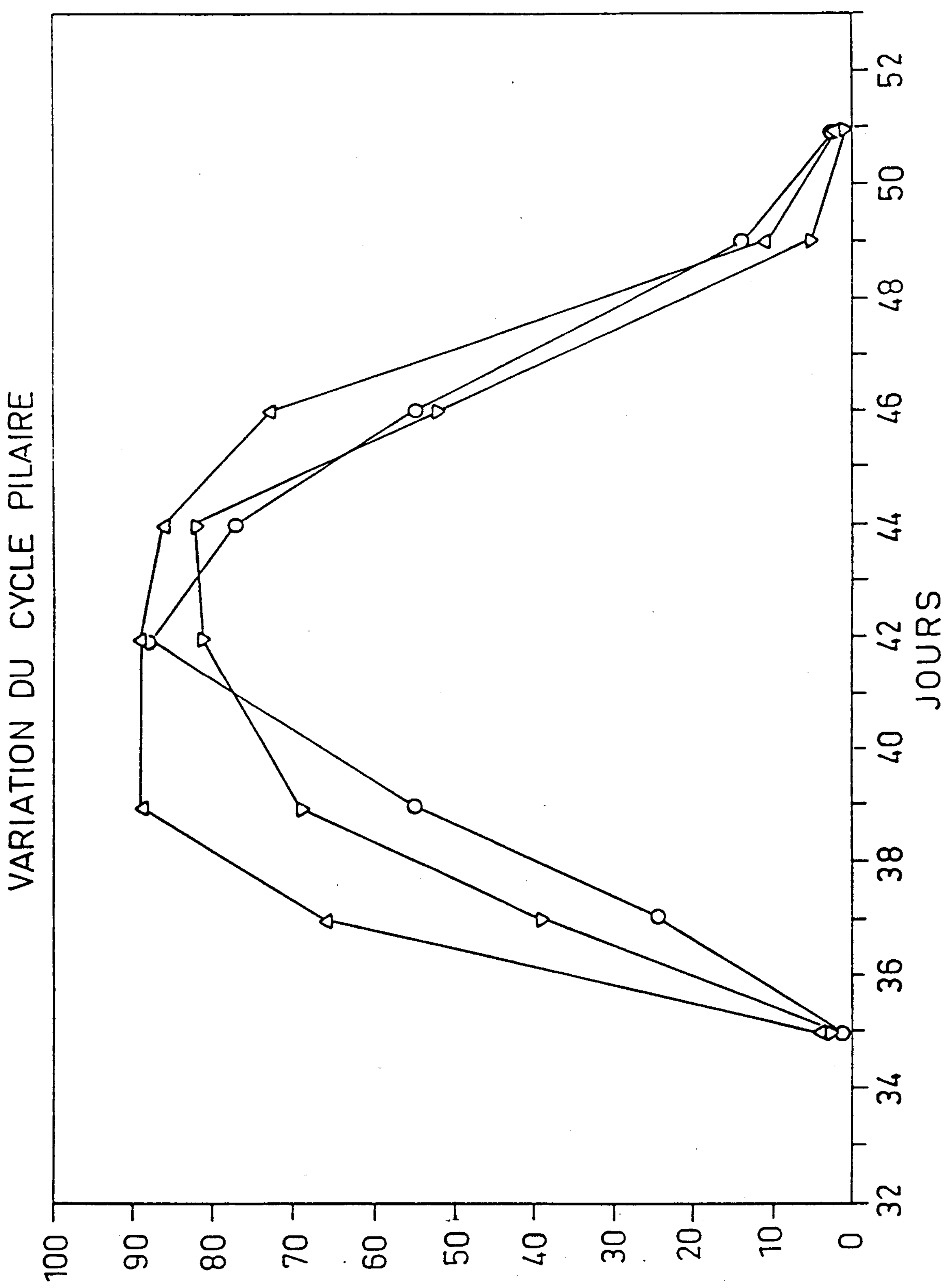
VARIATION DU CYCLE PILAIRE
% ANAGÈNE
JOURS
0
10
20
30
40
50
60
70
80
90
100
32
34
36
38
40
42
44
46
48
50
52